# EUROPEAN PATENT SPECIFICATION

(11) **EP 3 628 311 B1**
(45) Date of publication and mention of the grant of the patent: **11.11.2020**
(21) Application number: 18382687.4
(22) Date of filing: 27.09.2018
(51) Int. Cl.: A61K 9/48, A61K 9/50, A61K 31/4402, A61K 31/4415

(54) **A PROCESS FOR THE PREPARATION OF A MODIFIED RELEASE MULTIPLE UNIT ORAL DOSAGE FORM OF DOXYLAMINE SUCCINATE AND PYRIDOXINE HYDROCHLORIDE**
VERFAHREN ZUR HERSTELLUNG EINER ORALEN DARREICHUNGSFORM AUS MEHRFACHEN EINHEITEN MIT MODIFIZIERTER FREISETZUNG AUS DOXYLAMINSUCCINAT UND PYRIDOXINHYDROCHLORID
PROCÉDÉ DE PRÉPARATION D'UNE FORME POSOLOGIQUE ORALE À UNITÉS MULTIPLES ET À LIBÉRATION MODIFIÉE DE SUCCINATE DE DOXYLAMINE ET D'HYDROCHLORURE DE PYRIDOXINE

(43) Date of publication of application: 01.04.2020
(73) Proprietor: Inibsa Ginecologia, S.A., 08185 Lliça de Vall (ES)
(72) Inventor: Saura i Valls, Marc, 08024 BARCELONA (ES); Nebot Troyano, Joaquín, 08750 MOLINS DE REI (ES); Roca i Juanes, Ramon M., 08014 BARCELONA (ES)
(74) Representative: ZBM Patents - Zea, Barlocci & Markvardsen

(56) References cited:
- WO-A1-2016/029290
- WO-A1-2016/083278
- Inibsa Ginecología: "SPC Cariban 10mg/10mg modified release capsules", , March 2016 (2016-03), XP002789526, Retrieved from the Internet: URL:https://inibsa.com/wp-content/uploads/ 2016/10/En-spc-V1.pdf [retrieved on 2019-03-07]

## Description

The present invention relates to a process for the preparation of a modified release multiple unit oral dosage form of modified release pellets of doxylamine or a pharmaceutically acceptable salt thereof and modified release pellets of pyridoxine or a pharmaceutically acceptable salt thereof.

### Background Art

Doxylamine is the International Nonproprietary Name of (*RS*)-*N*,*N*-dimethyl-2-(1-phenyl-1-pyridin-2-yl-ethoxy)-ethanamine having the CAS number 469-21-6. Doxylamine is the first-generation of antihistamine that competitively, reversibly and non-specifically blocks H1 receptors and is also a non-specific antagonist that can block other receptors such as central or peripheral muscarinic receptors, with marked anticholinergic activity. It is commonly used in form of a salt and particularly in form of its succinate salt. The structure of doxylamine corresponds to the formula (I):

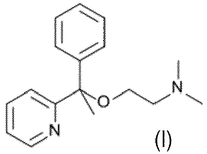

On the other hand, pyridoxine also known as vitamin B₆ is the International Nonproprietary Name of 4,5-Bis(hydroxymethyl)-2-methylpyridin-3-ol having the CAS number 65-23-6. Pyridoxine is a water-soluble vitamin factor whose active form is pyridoxal phosphate. It acts as an enzyme co-factor in numerous biochemical reactions involved in the digestive breakdown of proteins and amino acids and, to a lesser extent, lipids and carbohydrates. It is also involved in the metabolism of unsaturated fatty acids and it is also a coenzyme for transaminases and decarboxylases allowing the conversion of tryptophan into nicotinic acid. Pyridoxine is commonly used in form of a salt and particularly in form of its hydrochloride salt. The structure of pyridoxine corresponds to the formula (II):

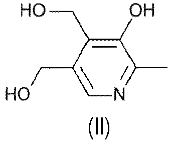

Doxylamine is commonly used by itself as a short-term sedative and also in combination with other drugs to provide night-time allergy and cold relief. Doxylamine is also used in combination with the analgesics paracetamol (acetaminophen) and codeine as an analgesic/calmative preparation, and is prescribed in combination with pyridoxine to prevent morning sickness in pregnant women.

Modified release oral dosage forms of doxylamine succinate and pyridoxine hydrochloride with different pharmacokinetic and pharmacological properties have been disclosed in the state of the art.

Several dual release oral dosage forms of doxylamine succinate and pyridoxine hydrochloride and processes for their preparation have been disclosed in the state of the art. These dual release oral dosage forms are formed by at least one immediate release composition and at least one modified release composition having each one of composition one or more of the active ingredients. This dosage system allows having an immediate release of one of the active ingredients and a modified release of the other active ingredients separately (cf. WO2013123569 and WO2016029290).

On the other hand, hard capsules filled with modified release pellets of doxylamine succinate and modified release pellets of pyridoxine hydrochloride are marketed with the name of Cariban. Cariban is used for the symptomatic treatment of nausea and vomiting. In particular, the effect of doxylamine and pyridoxine begins to be noted five hours after ingestion, which is advantageous because allows a prolonged therapeutic effect and a reduction of the drug intakes. However, it is not disclosed in the state of the art a process for its preparation.

The process used for the preparation of multiple unit oral dosage form (such as tablets or capsules having different plurality of pellets) determines the pharmacokinetic parameters of the active ingredients and also the therapeutic activity of the multiple unit dosage form. In particular, the therapeutic effect of the multiple unit dosage forms is conditioned by the dissolution profile of the active ingredients from each one of the pellets and also from the homogeneity inter-pellets.

Therefore, from what is known in the art it is derived that there is still the need of providing a cheap, robust, reproducible and scaling-up process for the preparation of homogeneous batches of a multiple unit oral dosage comprising modified release pellets of both active ingredients doxylamine and pyridoxine, exhibiting the appropriate dissolution profile for being used in therapy.

### Summary of Invention

Inventors have found that the process for the preparation of the present invention which comprises coating by spraying (coated) inert nucleus with a mixture of a modified release coating composition and simultaneously applying in solid form the active ingredient and/or a pore-forming agent (and optionally one or more pharmaceutically acceptable excipient or carrier) at an appropriate spray flow rate allows obtaining homogeneous batches of the multiple unit oral dosage form having modified release pellets of both active ingredients, doxylamine or a pharmaceutically acceptable salt thereof and pyridoxine or a pharmaceutically acceptable salt thereof, with the appropriate dissolution profile for being used in therapy with a prolonged therapeutic effect.

In particular, inventors have found that the process for the preparation of a modified release coating of the both plurality of pellets of the present invention allows that the multiple unit oral dosage form thus obtained exhibits the target dissolution profile, which means that:
from 10% to 35% by weight of doxylamine content is dissolved at 1^{st}h in 0.1 N HCI medium (pH = 1);
   then, the medium is replaced by a pH = 4.5 medium (0.05 M acetate buffer) and at 4^{th} h from an accumulated 45% to 70% by weight of doxylamine initial content is dissolved;
   then, the medium is replaced by a pH = 6.8 medium (0.05 M phosphate buffer) and at 7^{th} h at least an accumulated 80% of doxylamine initial content is dissolved
from 10% to 35% by weight of pyridoxine content is dissolved at 1^{st}h in 0.1 N HCI medium (pH = 1);
   then, the medium is replaced by a pH = 4.5 medium (0.05 M acetate buffer) and at 4^{th} h from an accumulated 40% to 65% by weight of pyridoxine initial content is dissolved;
   then, the medium is replaced by a pH = 6.8 medium (0.05 M phosphate buffer) and at 7^{th} h at least an accumulated 80% of pyridoxine initial content is dissolved.
wherein the dissolution profile is measured using a USP type II apparatus (basket), placing the composition in 900mL of the corresponding media / buffer at 37°C ±0.5 °C and 100 rpm.

Thus, the process for the preparation of a modified release coating of the both plurality of pellets of the present invention allows that the multiple unit oral dosage form thus obtained offers a modified sustained release of both active ingredients for at least 8 hours after administration. It is advantageous because the modified sustained release composition of the present invention has a dual effect, one immediate just after the administration and a prolonged release for the entire day, particularly after getting up.

Without being bound to any theory, it seems that the process of the invention allows obtaining a homogeneous type of pellets without the formation of aggregates and powdered mixtures which is advantageous for achieving the target dissolution profile.

Furthermore, the process of the invention also allows obtaining the both plurality of pellets with a high homogeneity of the content of active ingredient (i.e. a high inter-pellet homogeneity). It is advantageous because it means that each pellet has about the same amount of active ingredient and therefore, it assures that the multiple unit oral dosage form filled with those pellets always have the therapeutically effective amount of doxylamine or a pharmaceutically acceptable salt thereof, and pyridoxine or a pharmaceutically acceptable salt thereof. It means that infra- and supra dosification of active ingredients in the multiple unit oral dosage is avoided by using the process of the present invention.

Finally, inventors have also found that the process of the invention is also advantageous because it is cheaper, more robust and reproducible and ease to scale-up in comparison with the processes of the state of the art.

Thus, the aspect of the invention refers to a process for the preparation of a modified release multiple unit oral dosage form comprising:
a first plurality of modified release pellets of doxylamine or a pharmaceutically acceptable salt thereof comprising:
   - an inert nucleus;
   - an inner active coating layer comprising a therapeutically effective amount of doxylamine or a pharmaceutically acceptable salt thereof, one or more coating agents and one or more pore-forming agent;
and optionally one or more pharmaceutically acceptable excipients;
   - an intermediate enteric release coating layer comprising one or more enteric coating agents and one or more pore-forming agent; and
   - an external modified release coating layer comprising one or more enteric coating agents, one or more modified release coating agents and one or more pore-forming agent;
   and
a second plurality of modified release pellets of pyridoxine or a pharmaceutically acceptable salt thereof comprising:
   - an inert nucleus;
   - an inner active coating layer comprising a therapeutically effective amount of pyridoxine or a pharmaceutically acceptable salt thereof, and one or more coating agents; and
   - an external modified release coating layer comprising one or more enteric coating agents, one or more modified release coating agents and one or more pore-forming agents;
wherein the process comprises:
   - preparing the first plurality of modified release pellets of doxylamine or a pharmaceutically acceptable salt thereof by coating the pellets of doxylamine or a pharmaceutically acceptable salt thereof having the inner active coating layer and the intermediate enteric coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus; and the relation between the spray flow rate of the mixture comprising the coating agents and the addition rate of the pore-forming agents in solid form is from 90:10 to 60:40;
      and
   - preparing the second plurality of modified release pellets of pyridoxine or a pharmaceutically acceptable salt thereof by coating the pellets of pyridoxine or a pharmaceutically acceptable salt thereof having the inner active coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus; and the relation between the spray flow rate of the mixture comprising the coating agents and the addition rate of the pore-forming agents in solid form is from 90:10 to 60:40.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range. Ranges and values given, such as temperatures, times, and the like, should be considered approximate, unless specifically stated.

Furthermore, the terms "relation" and "relationship" have the same meaning and are used interchangeable. This term is used in the present invention for having the relation between the spray flow rate of the mixture comprising the coating agents and the addition rate of the pore-forming agents in solid form is from 90:10 to 60:40.

The process of the invention is a process for the preparation of a modified release multiple units oral dosage form containing a plurality of modified release pellets of doxylamine or a pharmaceutically acceptable salt thereof, and modified release pellets of pyridoxine or a pharmaceutically acceptable salt thereof. The term "multiple unit dosage form" defines a dosage form which consists of more than one unit which contains the effective amount of doxylamine and pyridoxine. Usually the multiple unit dosage forms are based on subunits such as granules, pellets or minitablets. They are usually delivered in hard gelatine capsules or transformed into tablets. In an embodiment, the multiple unit dosage form is a hard capsule filled with pellets as subunits having the active ingredient.

As it is disclosed above, the process of the invention prepare multiple unit dosage form including two plurality of pellets. The term "pellet" refers to small particles with approximately uniform shapes and sizes produced by an extrusion process or by layer-coating of inert nucleus. A "small particle" refers to a particle of which diameter, length, height, width, or the like is from 100 µm to 3000 µm, particularly from 300 µm to 2000 µm; more particularly from 600 µm to 1400 µm. Small particles have approximately uniform sizes if the diameter, length, height, width, or the like of the smallest particle is at least about one half of the average diameter, length, height, width, or the like of the particles and if the diameter, length, height, width, or the like of the largest particle is at most about twice the average diameter, length, height, width, or the like of the particles. Then, the term "pellet", "spherical pellet", "beads", "beadlets", "spherical particles", "spheroids" and "microspheres" have the same meaning and are used interchangeable. The term "granule" refers to small particles without approximately uniform shapes and sizes obtained by a granulation process. Generally granules are less uniform in size or shape than pellets. Thus, granules have a lower uniformity due to their irregular surfaces, and afford unacceptable dose uniformity and an inappropriate dissolution profile. Therefore, for the purpose of the invention the term "pellet" and "granule" are not the same and they are not interchangeable.

The term "modified release" dosage form and "modified delivery dosage form" as well as "modified release" pellet and "modified delivery" pellet have the same meaning and are interchangeable. Both terms are to be understood as a dosage form or pellet that exhibits a slower release of the active agents than that of a conventional immediate release pharmaceutical composition administered by the same route. In general, the term "modified release" refers that the active ingredient is released from the pharmaceutical dosage form in a controlled, sustained, prolonged or extended release.

In the context of the invention, the term "coating agents" and "film-forming agent coatings" have the same meaning and are used interchangeable. Both terms are to be understood as an agent capable of forming a thin polymer-based coat to a solid dosage form such as tablet and pellets. Examples of each one of the types of coating agents are disclosed below.

In particular, the term "modified release coating agent" refers to an agent capable of forming films which allow the delivery of the drug at a predetermined rate and/or location according to the needs of the body and disease states for a definite time of period. Illustrative but non-limitative examples of "modified release polymer" and "modified delivery polymer" are polymers which provide a controlled release, a sustained release, a prolonged release or an extended release. Examples of modified release coating agent include without limitation acrylic polymers, celluloses and their derivatives, shellac, zein, hydrogenated vegetable oil, hydrogenated castor oil, and their mixtures. Examples of suitable acrylic polymers include, but are not limited to, acrylic acid and methacrylic acid copolymers, methyl methacrylate copolymers, ethoxyethyl methacrylates, cyanoethyl methacrylate, aminoalkyl methacrylate copolymer, poly (acrylic acid), poly (methacrylic acid), methacrylic acid alkylamide copolymer, poly (methyl methacrylate), poly (methacrylic acid anhydride), methyl methacrylate, polymethacrylate, poly (methyl methacrylate) copolymer, polyacrylamide, aminoalkyl methacrylate copolymer, glycidyl methacrylate copolymers, shellac, and their mixtures. A modified release polymer is selected from the group consisting of acrylic acid and methacrylic acid copolymers and shellac. Preferably, the modified release polymer is shellac. The modified release polymers can be accompanied by plasticizers such as triethylcitrate (TEC), polyethylene glycol (PEG), cetyl and stearyl alcohol; surface-active agents such as sodium lauryl sulphate, polysorbate and poloxamer; pigments such as titanium dioxide, iron sesquioxide; lubricants such as talc, magnesium stearate or glyceril monostearate, and mixtures thereof.

The term "binder" refers to substances incorporated into formulations to facilitate the agglomeration of powder into granules or pellets during mixing with a granulating fluid such as water, hydroalcoholic mixtures, or other solvents. Examples of binding agents or binders include without limitation microcrystalline cellulose, hydroxypropyl methyl cellulose (HPMC), polyvinyl pyrrolidone (PVP), polyethylene glycol (PEG) 6000, guar gum, starch or shellac.

The term "enteric release" refers to a composition or layer of a dosage form that is formulated to release the active ingredient(s) upon exposure to a characteristic aspect of the gastrointestinal tract. In an embodiment, the enteric material is pH-sensitive and is affected by changes in pH encountered within the gastrointestinal tract (pH-sensitive release). The enteric material typically remains insoluble at gastric pH, then allows for release of the active ingredient in the higher pH environment of the downstream gastrointestinal tract (e.g. often the duodenum, or sometimes the colon). In another embodiment, the enteric material comprises enzymatically degradable polymers that are degraded by bacterial enzymes present in the lower gastrointestinal tract, particularly in the colon. Optionally, the unit dosage form is formulated with a pH-sensitive enteric material designed to result in a release within about appropriate hours when at or above a specific pH. In various embodiments, the specific pH can for example be from about 4 to about 7, such as about 4.5, 5, 5.5, 6, 6.5, 6.8 or 7. In the context of the invention, the term "enteric release coating agent" refers to an agent capable of forming films which allow the delivery of doxylamine and pyridoxine upon exposure to a characteristic aspect of the gastrointestinal tract as defined above. Materials used for enteric release formulations, for example as coatings, are well known in the art and include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the trade-name Acryl-EZE® (Colorcon, USA), Eudragit® (Rohm Pharma; Westerstadt, Germany), including Eudragit® L30D-55 and L100-55 (soluble at pH 5.5 and above), Eudragit® L100 and L12,5 (soluble at pH 6.0 and above), Eudragit® S, S12,5 and FS 30D (soluble at pH 7.0 and above, as a result of a higher degree of esterification), and Eudragits® NE, NM,RL and RS (water- insoluble polymers having different degrees of permeability and expandability); vinyl polymers and copolymers such as polyvinyl pyrrolidone, vinyl acetate, vinyl acetate phthalate, vinyl acetate crotonic acid copolymer, and ethylene-vinyl acetate copolymer; enzymatically degradable polymers such as azo polymers, pectin, chitosan, amylose and guar gum; zein and shellac. Combinations of different enteric materials may also be used. Multi-layer coatings using different polymers may also be applied. The properties, manufacture and design of enteric delivery systems are well known to those of ordinary skill in the art. In a particular embodiment, the enteric coating agent is selected from the group consisting of copolymer of methacrylic acid and methyl methacrylate, copolymer of methacrylic acid and methyl acrylate, cellulose acetate phthalate, hydroxypropyl methyl cellulose phthalate, polyvinyl acetate phthalate sodium alginate, cellulose acetate trimellitate and a mixture thereof. More particularly, Eudragit L® such as for example Eudragit L100 (sold by Evonic).

As it is defined above, the process of the present invention comprises preparing a first plurality of pellets which comprises a therapeutically effective amount of doxylamine or a pharmaceutically acceptable salt thereof and a second plurality of pellets which comprises a therapeutically effective amount of pyridoxine or a pharmaceutically acceptable salt thereof.

The term "therapeutically effective amount" as used herein, refers to the amount of an active ingredient per multiple unit dosage form that, when administered, is sufficient to prevent development of, or alleviate to some extent, one or more of the symptoms of the disease which is addressed. The particular dose of compound administered according to this invention will be determined by the particular circumstances surrounding the case, including the compound administered, the route of administration, the particular condition being treated, and the similar considerations.

The term "pharmaceutically acceptable salt(s)" used herein encompasses any salt formed from pharmaceutically acceptable non-toxic acids including inorganic or organic acids. There is no limitation regarding the salts, except that if used for therapeutic purposes, they must be pharmaceutically acceptable. As doxylamine and pyridoxine are basic compounds, salts may be prepared from pharmaceutically acceptable non-toxic acids, including inorganic and organic acids. Such acids include among others acetic, benzene sulfonic, benzoic, camphor sulfonic, citric, ethansulfonic, fumaric, gluconic, glutamic, hydrobromic, hydrochloric, lactic, maleic, malic, mandelic, methanesulfonic, phosphoric, succinic, sulphuric, tartaric or p-toluensulfonic acid.

The preparation of pharmaceutically acceptable salts of doxylamine and pyridoxine can be carried out by methods known in the art. For instance, they can be prepared from the parent compound, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them.
In an embodiment, the process comprises preparing a first plurality of pellets which comprises a therapeutically effective amount of a pharmaceutically acceptable salt of doxylamine, particularly succinate salt of doxylamine. In an embodiment, the process comprises preparing a multiple unit dosage form having the first plurality of pellets which comprises from 5 mg to 50 mg of doxylamine succinate per multiple unit dosage form, particularly from 6 mg to 40 mg, from 7 mg to 30 mg, from 8 mg to 20 mg, and more particularly from 9 mg to 11 mg of doxylamine succinate. In a particular embodiment, the process comprises preparing a multiple unit dosage forms having the first plurality of pellets having 10 mg of doxylamine succinate per multiple unit dosage form. In an embodiment, the process comprises preparing a second plurality of pellets which comprises a therapeutically effective amount of a pharmaceutically acceptable salt of pyridoxine, particularly pyridoxine hydrochloride. In an embodiment, the process comprises preparing a multiple unit dosage form having the second plurality of pellets which comprises from 5 mg to 50 mg of pyridoxine hydrochloride per multiple unit dosage form, particularly from 6 mg to 40 mg, from 7 mg to 30 mg, from 8 mg to 20 mg, and more particularly from 9 mg to 11 mg of pyridoxine hydrochloride. In a particular embodiment, the process comprises preparing a multiple unit dosage forms having the first plurality of pellets having 10 mg of pyridoxine hydrochloride per multiple unit dosage form. In an embodiment, the process comprises preparing a multiple unit dosage form comprising the first plurality of pellets which comprises a therapeutically effective amount of a pharmaceutically acceptable salt of doxylamine, particularly succinate salt of doxylamine; and the second plurality of pellets which comprises a therapeutically effective amount of a pharmaceutically acceptable salt of pyridoxine, particularly pyridoxine hydrochloride. In an embodiment, the process comprises preparing a multiple unit dosage form comprising the first plurality of pellets which comprises from 5 mg to 50 mg of doxylamine succinate per multiple unit dosage form, particularly 10 mg of doxylamine succinate per multiple unit dosage form; and the second plurality of pellets which comprises from 5 mg to 50 mg of pyridoxine hydrochloride per multiple unit dosage form, particularly 10 mg of pyridoxine hydrochloride per multiple unit dosage form. In a particular embodiment, the process comprises preparing the first plurality of pellets which comprises 10 mg of doxylamine succinate per multiple unit dosage form and the second plurality of pellets comprising 10 mg of pyridoxine hydrochloride per multiple unit dosage form.

In an embodiment, the process comprises preparing a multiple unit dosage form comprising from 20 mg to 220 mg of the first plurality of pellets which comprises a therapeutically effective amount of a pharmaceutically acceptable salt of doxylamine, particularly succinate salt of doxylamine; and from 20 mg to 220 mg of the second plurality of pellets which comprises a therapeutically effective amount of a pharmaceutically acceptable salt of pyridoxine, particularly pyridoxine hydrochloride. In an embodiment, the process comprises preparing a multiple unit dosage form comprising from 20 mg to 220 mg, particularly 60mg, of the first plurality of pellets which comprises from 5 mg to 50 mg of doxylamine succinate, particularly 10 mg of doxylamine succinate; and from 20 to 220 mg, particularly 60mg, of the second plurality of pellets which comprises from 5 mg to 50 mg of pyridoxine hydrochloride, particularly 10 mg of pyridoxine hydrochloride. In an embodiment, the process comprises preparing a multiple unit dosage form comprising the first plurality of pellets which comprises from 5 mg to 50 mg of doxylamine succinate, particularly 10 mg of doxylamine succinate; and the second plurality of pellets which comprises from 5 mg to 50 mg of pyridoxine hydrochloride, particularly 10 mg of pyridoxine hydrochloride. In a particular embodiment, the process comprises preparing the first plurality of pellets which comprises 10 mg of doxylamine succinate and the second plurality of pellets comprising 10 mg of pyridoxine hydrochloride.

In an embodiment, the process comprises preparing a multiple unit dosage form comprising the first plurality of pellets which comprises a therapeutically effective amount of a pharmaceutically acceptable salt of doxylamine having a particle size from 1 µm to 250 µm; particularly from 1 µm to 150 µm; more particularly lower than 100 µm. In an embodiment, the process is one wherein the doxylamine or a salt thereof; particularly doxylamine succinate has a D90 particle diameter from 1 µm to 250 µm; particularly from 1 µm to 150 µm; more particularly lower than 100 µm.

The selection of size ranges or size fractions of particles by sieving is well known. One standard way of defining the particle size distribution in a sample of particles is to refer to D10, D50 and D90 values. D10 is the particle diameter values that 10% of the population of particles lies below. D50 is the particle diameter values that 50 % of the population lies below and 50% of the population lies above. D50 is also known as the median particle size value. D90 is the particle diameter values that 90 % of the population lies below.

In an embodiment, the process comprises preparing a multiple unit dosage form comprising the first plurality of pellets which comprises a therapeutically effective amount of a pharmaceutically acceptable salt of pyridoxine having a particle size equal to or lower than 500 µm. In an embodiment, the process is one wherein the pyridoxine or a salt thereof; particularly pyridoxine hydrochloride has a particle size from 1 µm to 250 µm; particularly from 1 µm to 150 µm; more particularly lower than 100 µm.

The particle size of the active ingredients can be measured by any method disclosed in the state of the art. Examples of methods commonly used for measuring the particle size is Dynamic light scattering (DLS) reporting the number average diameter value, Atomic force microscopy (AFM) or transmission electron microscopy (TEM) to measure dried particles; laser diffraction (Laser Masterizer, Mie Theory; ISO 13320-1) and by sedimentation analysis (Sedigraph-Stoke's Law; ISO 13317-3).

The term "pore-forming agent" refers to any agent capable of forming one or more pores in the shell/coating to allow modified the release of the active ingredients. The pore-forming agent can be organic or inorganic, or any combination thereof. Examples of pore-forming agent include, but are not limited to, talc, polyethylene glycol (PEG), propylene glycol, isopropyl alcohol, glycerol, lactose, glucose, sucrose, mannitol, sorbitol, sodium chloride, potassium chloride, hydroxypropyl cellulose, micronized sugar, hydroxypropyl methyl cellulose (HPMC), polyvinyl alcohols, methacrylic acid copolymers, or a mixture therefore. In an embodiment, the pore-forming agent is selected from the group consisting of talc, micronized sugar, sodium or potassium chloride and a mixture thereof. In an embodiment, the process is one wherein the pore-forming agent has a D90 particle diameter equal to or lower than 250 µm; particularly lower than 150 µm; more particularly lower than 100 µm. In a particular embodiment, the process is one wherein the pore-forming agent is talc having a D90 particle diameter lower than 75 µm.

The particle size of the pore-forming agent can be measured by any method disclosed in the state of the art. In particular, the method used in the present invention for measuring the particle size is by laser diffraction (Laser Masterizer, Mie Theory; ISO 13320-1) and by sedimentation analysis (Sedigraph-Stoke's Law; ISO 13317-3)

The term "inert nucleus" refers to neutral microspheres which can have in their composition one or more of the following substances: sorbitol, mannitol, saccharose, starch, microcrystalline cellulose, lactose, glucose, trehalose, maltitol or fructose. The initial size of this inert nucleus can be between 200 and 1800 micrometres. In an embodiment, the inert nucleuses are neutral microspheres of a mixture of sucrose and starch.

In an embodiment, the process comprises preparing a multiple unit dosage form which can comprise one or more pharmaceutically acceptable excipients or carriers. The term "pharmaceutically acceptable excipients or carriers" refers to that excipients or carriers suitable for use in the pharmaceutical technology for preparing compositions with medical use. The appropriate excipients and/or carriers, and their amounts, can readily be determined by those skilled in the art according to the type of formulation being prepared. In an embodiment, the modified release multiple unit dosage form of the present invention further comprises a binder, an alkaline agent, a glidant, a surfactant, or a mixture thereof.

As it is mentioned above, the process of the invention comprises coating separately the pellets of doxylamine or a pharmaceutically acceptable salt thereof having the active layer and the intermediate enteric coating layer; and the pellets of pyridoxine or the pharmaceutically acceptable salt thereof having the inner active coating layer, wherein the modified release coating step comprising: simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40. It means that the process is performed by spraying the mixture having the coating agents and at the same time adding the ingredients that are in powder form. In an embodiment, the process is one wherein the mixture comprising the enteric coating agents and the modified release coating agents as defined in the present invention has a weight ratio from 5:95 to 30:70. In an embodiment, the process is one as defined above wherein the mixture comprises methacrylic acid-methyl methacrylate copolymer (1:1) (Eudragit L) as enteric coating agent and (dewaxed) shellac as modified release coating agent in a weight ratio from 5:95 to 30:70; particularly from 8:92 to 20:80. In an embodiment, the process is one wherein the spray flow rate of the mixture comprising the coating agents is from 300 to 1200 mg/min per Kg of inert nucleus; particularly from 385 to 1000g/min per Kg of inert nucleus.

In an embodiment, the process of the invention comprises coating separately the pellets of doxylamine or a pharmaceutically acceptable salt thereof having the active layer and the intermediate enteric coating layer as defined above; then the process is one wherein the solid addition rate of the pore-forming agents is from 88 to 195 mg/min per Kg of inert nucleus. In an embodiment, the process is one wherein the relation between the spray flow rate of the mixture comprising the coating agents and the solid addition rate of the pore-forming agents is from 90:10 to 60:40; particularly from 90:10 to 70:30. In an embodiment, the process is one wherein the mixture comprising from 2.5 to 7.5% of the enteric coating agents and from 15 to 30% of the modified release coating agents is a solution further comprising one or more organic solvents; particularly in an amount from 85 to 290 g of solvent per Kg of inert nucleus; particularly from 250 to 290 g of solvent per Kg of inert nucleus; more particularly from 270 to 285 g of solvent per Kg of inert nucleus

In an embodiment, the process of the invention comprises coating separately the pellets of pyridoxine or the pharmaceutically acceptable salt thereof having the inner active coating layer, then the process is one wherein the solid addition rate of the pore-forming agents is from 193 to 425 mg/min per Kg of inert nucleus. In an embodiment, the process is one wherein the relation between the spray flow rate of the mixture comprising the coating agents and the solid addition rate of the pore-forming agents is from 90:10 to 60:40; particularly from 80:20 to 60:40. In an embodiment, the process is one wherein the mixture comprises from 2.5 to 5.5% of the enteric coating agents and from 20 to 35% of the modified release coating agents is a solution further comprising one or more organic solvents; particularly in an amount from 85 to 100 g of solvent per Kg of inert nucleus; particularly from 90 to 95 g of solvent per Kg of inert nucleus; more particularly from 280 to 290 g of solvent per Kg of inert nucleus.

In an embodiment, the process of the invention comprises coating separately the pellets of doxylamine or a pharmaceutically acceptable salt thereof having the active layer and the intermediate enteric coating layer as defined above; or the pellets of pyridoxine or the pharmaceutically acceptable salt thereof having the inner active coating layer, is one wherein the mixture comprising the enteric coating agents and the modified release coating agents is a solution further comprising one or more organic solvents selected from the group consisting of (C₁-C₄)alcohol, (C₁-C₄)alkyl-CO-(C₁-C₄)alkyl, (C₁-C₄)alkyl-CO-O-(C₁-C₄)alkyl, water and mixtures thereof. The term "alcohol" refers to an "alkane" wherein at least one hydrogen atom is substituted by a hydroxyl group and which contains the number of carbon atoms specified in the description or claims. The term "alkane" refers to a saturated, branched or linear hydrocarbon which contains the number of carbon atoms specified in the description or claims. Examples include methanol, ethanol, n-propanol, iso-propanol, butanol, iso-butanol, and sec-butanol. The term "alkyl" refers to a saturated straight, or branched hydrocarbon chain which contains the number of carbon atoms specified in the description or claims. Examples include, among others, the group methyl, ethyl, propyl, isopropyl, butyl, isobutyl, sec-butyl, and tert-butyl. In an embodiment, the process is one wherein the mixture comprising the enteric coating agents and the modified release coating agents is a solution further comprising one or more organic solvents selected from the group consisting of ethanol, 2-propanol, methanol, acetone, butanone, ethyl acetate, water and a mixture thereof; particularly selected from ethanol acetone, water and a mixture thereof.

In an embodiment, the process of the invention comprises coating separately the pellets of doxylamine or a pharmaceutically acceptable salt thereof having the active layer and the intermediate enteric coating layer; and the pellets of pyridoxine or the pharmaceutically acceptable salt thereof having the inner active coating layer, wherein the airflow is from 0 a 20 m³/h per Kg of inert nucleus; particularly from 0 to 4 m3 /h per Kg of inert nucleus. The airflow is controlled using an anemometer type air inlet detection system installed in the duct between the fan and the coating pan, or using another equivalent system.

In an embodiment, the process of the invention comprises coating separately the pellets of doxylamine or a pharmaceutically acceptable salt thereof having the active layer and the intermediate enteric coating layer; and the pellets of pyridoxine or the pharmaceutically acceptable salt thereof having the inner active coating layer, wherein the core temperature of the pellets during the coating step is from 5°C to 50 °C; particularly from 10°C to 30°C; more particularly from 15°C to 25 °C. The temperature is controlled using a calibrated PT100 sensor being directly in contact with the pellets being coated, but it could be controlled using an equivalent system.

In an embodiment, the process of the invention comprises coating separately the pellets of doxylamine or a pharmaceutically acceptable salt thereof having the inner active coating layer and the intermediate enteric coating layer; and the pellets of pyridoxine or the pharmaceutically acceptable salt thereof having the inner active coating layer, wherein the rotation speed is from 0 to 50 rpm, particularly from 2 to 25 rpm; more particularly from 10 to 20 rpm. The rotation pan speed can be controlled by any method known in the state of the art. In particular, the method used in the present invention is by using a revolution counter.

In an embodiment, wherein the process of the invention comprises coating the pellets of doxylamine or a pharmaceutically acceptable salt thereof having the inner active coating layer and the intermediate enteric coating layer; then the coating step comprising: spraying a mixture comprising from 2.0 to 7.5 % by weight of the enteric coating agents and from 15% to 35 % by weight of the modified release coating agents; wherein the weight ratio between the enteric coating agents and the modified release coating agents is from 15:85 to 25:75. Particularly, the mixture comprises from 4 to 7% by weight of the methacrylic acid-methyl methacrylate copolymer (1:1) (Eudragit L) as enteric coating agent and from 15 to 25 % by weight of (dewaxed) shellac as modified release coating agent in a weight ratio from 15:85 to 25:75.

In an embodiment, wherein the process of the invention comprises coating the pellets of pyridoxine or the pharmaceutically acceptable salt thereof having the inner active coating layer; then the coating step comprising: spraying the mixture comprising from 2.0 to 7.5% by weight of the enteric coating agents and from 15 to 35%, particularly from 20 to 35%, of the modified release coating agents wherein the weight ratio between the enteric coating agents and the modified release coating agents is from 5:95 to 30:70. Particularly, the mixture comprises from 2.0 to 5% by weight of methacrylic acid-methyl methacrylate copolymer (1:1) (Eudragit L) as enteric coating agent and from 25 to 35% by weight of (dewaxed) shellac as modified release coating agent in a weight ratio from 5:95 to 15:85.

In an embodiment, wherein the process of the invention comprises coating the pellets of doxylamine or a pharmaceutically acceptable salt thereof having the inner active coating layer and the intermediate enteric coating layer; then the coating step comprising simultaneously spraying a mixture comprising from 2.0 to 7.5% by weight of the enteric coating agents and from 15 to 35% by weight of the modified release coating agents in a weight ratio from 5:95 to 30:70; and adding the pore-forming agents in powder form, wherein: the spray flow rate of the mixture comprising the coating agents is from 300 to 1200 mg/min per Kg of inert nucleus; particularly from 445 to 1000 g/min per Kg of inert nucleus; more particularly about 890 mg/min kg pellets; the solid addition rate of the pore-forming agents is from 88 to 195 mg/min per Kg of inert nucleus, more particularly 178 mg/min per kg pellets; and the relation between the spray flow rate of the mixture comprising the coating agents and the solid addition rate of the pore-forming agents is from 90:10 to70:30, particularly from 85:15 to 75:25, more particularly 83:17. It means that the process is performed by spraying the mixture having the coating agents and at the same time adding the ingredients that are in powder form. In an embodiment, the process is one wherein the mixture comprising the enteric coating agents and the modified release coating agents as defined in the present invention has a weight ratio from 10:90 to 30:70, particularly from 15:85 to 25:75. In an embodiment, the process is one wherein the mixture comprises methacrylic acid-methyl methacrylate copolymer (1:1) (Eudragit L) as enteric coating agent and (dewaxed) shellac as modified release coating agent in a weight ratio from 15:85 to 25:75; particularly from 18:82 to 22:78 and more particularly 20:80. In an embodiment, the process is one wherein the spray flow rate of the mixture comprising the coating agents is from 300 to 1200 mg/min per Kg of inert nucleus; particularly from 445 to 1000 mg/min per Kg of inert nucleus, more particularly 890 mg/min per Kg pellets. In an embodiment, the process is one wherein the solid addition rate of the pore-forming agents is from 88 to 195 mg/min per Kg of inert nucleus, more particularly 178 mg/min per kg pellets. In an embodiment, the process is one wherein the relation between the spray flow rate of the mixture comprising the coating agents and the solid addition rate of the pore-forming agents from 85:15 to 75:25, particularly 83:17. In an embodiment, the process is one wherein the mixture comprising from 2 to 7.5% by weight of the enteric coating agents are as defined above, particularly is methacrylic acid-methyl methacrylate copolymer (Eudragit L), and from 15 to 35% by weight of the modified release coating agents is a solution further comprising one or more organic solvents; particularly in an amount of solvents from 275 to 290 g/min per Kg of pellet, more particularly 282 g/kg pellet, having the mixture an overall solvent concentration from 72 to 78% by weigh. In an embodiment, the process is one wherein the mixture comprising the enteric coating agents and the modified release coating agents is a solution further comprising one or more organic solvents as defined above.

In an embodiment, wherein the process of the invention comprises coating the pellets of pyridoxine or the pharmaceutically acceptable salt thereof having the inner active coating layer; then the coating step comprising: simultaneous spraying a mixture comprising from 2.0 to 7.5 %; particularly from 2 to 5.5%, by weight of the enteric coating agents and from 15 to 35 % by weight of the modified release coating agents in a weight ratio from 5:95 to 25:75; and adding the pore-forming agents in powder form, wherein: the spray flow rate of the mixture comprising the coating agents is from 385 to 850 mg/min per Kg of inert nucleus; the solid addition rate of the pore-forming agents is from 193-to 425 mg/min per Kg of inert nucleus; and the relation between the spray flow rate of the mixture comprising the coating agents and the solid addition rate of the pore-forming agents is from 80:20 to 60:40. It means that the process is performed by spraying the mixture having the coating agents and at the same time adding the ingredients that are in powder form. In an embodiment, the process is one wherein the mixture comprising the enteric coating agents and the modified release coating agents as defined in the present invention has a weight ratio from 5:95 to 30:70. In an embodiment, the process is one wherein the mixture comprises methacrylic acid-methyl methacrylate copolymer (1:1) (Eudragit L) as enteric coating agent and (dewaxed) shellac as modified release coating agent in a weight ratio from 5:95 to 25:75; particularly from 5:95 to 15:85 and more particularly 9:91. In an embodiment, the process is one wherein the spray flow rate of the mixture comprising the coating agents is from 385 to 850 mg/min per Kg of inert nucleus; particularly 773 mg/min per Kg of inert nucleus. In an embodiment, the process is one wherein the solid addition rate of the pore-forming agents; particularly talc, is from 193 to 425 mg/min per Kg of inert nucleus; particularly 387 mg/min per Kg of inert nucleus. In an embodiment, the process is one wherein the relation between the spray flow rate of the mixture comprising the coating agents and the solid addition rate of the pore-forming agents is from 90:10 to 60:40; particularly from 75:25 to 65:35, more particularly 67:33. In an embodiment, the process is one wherein the mixture comprising from 2.0 to 7.5% of the enteric coating agents and from 15 to 35% of the modified release coating agents is a solution further comprising one or more organic solvents; particularly in an overall amount of solvents from 90 to 95 g per Kg of pellet, more particularly 93 g/kg pellet, having the mixture an overall solvent concentration from 65 to 70% by weigh, more particularly a 68% by weight of solvents. In an embodiment, the process is one wherein the mixture comprising the enteric coating agents and the modified release coating agents is a solution further comprising one or more organic solvents as defined above.

In an embodiment, the process of the invention further comprises a previous step of coating separately the inert nucleus with a simultaneous spraying of a mixture comprising from 30 to 40% by weight one or more coating agents and adding a powdered mixture of the therapeutically effective amount of doxylamine or a pharmaceutically acceptable salt thereof, particularly doxylamine succinate, from 22 to 30% by weight of the pore-forming agents, and optionally one or more pharmaceutically acceptable excipients; wherein the spray flow rate of the mixture comprising from 30 to 40% by weight one or more coating agents is from 445 to 1000 mg/min per Kg of inert nucleus; the solid addition rate of the powdered mixture is from 1,64 to 3,62 g/min per Kg of inert nucleus; and the relation between the spray flow rate of the mixture comprising the coating agents and the solid addition rate of the powdered mixture is from 15:85 to 25:75. In an embodiment, the process is one wherein the mixture comprising from 30 to 40% by weight of one or more of the coating agents is a solution further comprising one or more organic solvents; particularly in an amount from 60 to 70% by weight. In an embodiment, the process is one wherein the mixture comprising the coating agents is a solution further comprising one or more organic solvents selected from the group consisting of ethanol, 2-propanol, methanol, acetone, butanone, ethyl acetate and water or a mixture thereof.

In an embodiment, the process of the invention further comprises a previous step of coating separately the inert nucleus with a simultaneous spraying of a mixture comprising from 30 to 40% by weight one or more coating agents and adding a powdered mixture of the therapeutically effective amount of doxylamine or a pharmaceutically acceptable salt thereof, particularly doxylamine succinate, from 22 to 30% by weight of the pore-forming agents, and optionally one or more pharmaceutically acceptable excipients; wherein the spray flow rate of the mixture comprising from 30 to 40% by weight one or more coating agents is from 445 to 1000 mg/min per Kg of inert nucleus; the solid addition rate of the powdered mixture is from 1.64 to 3.62 g/min per Kg of inert nucleus; and the relation between the spray flow rate of the mixture comprising the coating agents and the solid addition rate of the powdered mixture is from 15:85 to 25:75; wherein the coating agent is selected from the group consisting of polyvinylpyrrolidone, shellac, hyproxypropylmethylcellulose, hydroxypropylcellulose, and microcrystalline cellulose and mixture thereof; particularly a mixture of polyvinylpyrrolidone and shellac. Particularly, the mixture comprising from 30 to 40% by weight of one or more coating agents is a mixture of polyvinylpyrrolidone K30 and (dewaxed) shellac in a weight ratio from 10:90 to 18:82 of solid coating agents (this values are equivalent to a weight ratio from polyvinylpyrrolidone and shellac from 20:80 to 30:70). More particularly, a mixture of a solution of polyvinylpyrrolidone K-30 20% in ethanol and a solution of (dewaxed) shellac 40% w/w in ethanol in a weight ratio from 20:80 to 40:60; particularly 30:70.

In an embodiment, the process of the invention further comprises a previous step of coating separately the inert nucleus with a simultaneous spraying of a mixture comprising from 30 to 40 % by weight of one or more coating agents and adding a powdered mixture of the therapeutically effective amount of doxylamine or a pharmaceutically acceptable salt thereof, particularly doxylamine succinate, from 22 to 30% by weight of the pore-forming agents, and optionally one or more pharmaceutically acceptable excipients; wherein the spray flow rate of the mixture comprising from 30 to 40% by weight one or more coating agents is from 445 to 1000 mg/min per Kg of inert nucleus; the solid addition rate of the powdered mixture is from 1.64 to 3.62 g/min per Kg of inert nucleus; and the relation between the spray flow rate of the mixture comprising the coating agents and the solid addition rate of the powdered mixture is from 15:85 to 30:70. In an embodiment, the process is one wherein the mixture comprising from 30 to 40% by weight of one or more of the coating agents is a solution further comprising one or more organic solvents; particularly in an overall amount of solvents from 61 to 71 g per Kg of inert nucleus, more particularly 66 g per Kg of inert nucleus, having the mixture an overall solvent concentration from 0 to 70% by weight. In an embodiment, the process is one wherein the pore-forming agent is selected from talc, micronized sugar, sodium or potassium chloride and a mixture thereof; particularly talc. In an embodiment, the process is one wherein the mixture comprising the coating agents is a solution further comprising one or more organic solvents as defined above. In an embodiment, the process of the invention further comprises a previous step of coating separately the inert nucleus with a simultaneous spraying of a mixture comprising from 30 to 40% by weight of one or more coating agents and adding a powdered mixture of the therapeutically effective amount of doxylamine or a pharmaceutically acceptable salt thereof, particularly doxylamine succinate, from 22 to 30% by weight of the pore-forming agents, and optionally one or more pharmaceutically acceptable excipients as defined above wherein the pharmaceutically acceptable excipient is selected from the group consisting of one or more glidant, fillers, wicking agents or mixtures thereof.

The term "glidant" refers to a substance which improves the flow characteristics of powder mixtures in the dry state. Materials commonly used as a glidant include magnesium stearate, colloidal silicon dioxide or talc. In an embodiment, the composition of the invention is one wherein the pharmaceutically acceptable excipients or carriers comprises one or more glidant; preferably comprises colloidal silicon dioxide, and more preferably Aerosil 200 Pharma.

The term "filler" refers to materials that are added to bulk-up a dosage form when the active ingredient is no present in sufficient quantity. Materials commonly used as filler include lactose, sucrose, mannitol, dicalcium phosphate dehydrate, starch, cellulose and microcrystalline cellulose. In an embodiment, the composition of the invention is one wherein the pharmaceutically acceptable excipients or carriers comprises one or more fillers; preferably comprises sucrose, starch or microcrystalline cellulose.

The term "wicking agents" refers to the material that has the ability to draw water into the porous network of a delivery device. It has the ability to undergo physiosorption with water. The role of a wicking agent is to act like a carrier and facilitate the entry of water to the inner surfaces of the core. Materials commonly used as wicking agent include sodium lauryl sulfate, kaolin, titanium dioxide, alumina, bentonite, magnesium aluminium silicate, povidone and colloidal silicon dioxide (Aerosil). In an embodiment, the composition of the invention is one wherein the pharmaceutically acceptable excipients or carriers comprises one or more wicking agents; preferably kaolin, titanium dioxide, alumina, bentonite, magnesium aluminium silicate, povidone and colloidal silicon dioxide (Aerosil). In an embodiment, the composition of the invention is one wherein the pharmaceutically acceptable excipients or carriers comprises one or more wicking agents; preferably comprises povidone or colloidal silicon dioxide (Aerosil) or a mixture thereof.

In an embodiment, the process of the invention further comprises a previous step of coating separately the inert nucleus with a simultaneous spraying of a mixture comprising from 30 to 40% by weight of one or more coating agents and adding a powdered mixture of the therapeutically effective amount of doxylamine or a pharmaceutically acceptable salt thereof, particularly doxylamine succinate, from 22 to 30% by weight of the pore-forming agents, and one or more pharmaceutically acceptable excipients as defined above, wherein the spray flow rate of the mixture comprising from 30 to 40 % by weight of one or more coating agents is from 445 to 1000 mg/min per Kg of inert nucleus; the spray flow of the powdered mixture is from 1,64 to 3,62 g/min per Kg of inert nucleus; and the relation between the spray flow rate of the mixture comprising the coating agents and the solid addition rate powdered mixture is from 15:85 to 30:70. In an embodiment, doxylamine or a pharmaceutically acceptable salt thereof, particularly doxylamine succinate, has a particle size equal to or lowers than 100 µm.

In an embodiment, the process of the invention further comprises a previous step of coating separately the inert nucleus with a simultaneous spraying of a mixture comprising from 30 to 45% of one or more coating agents and adding the therapeutically effective amount of pyridoxine or a pharmaceutically acceptable salt thereof; particularly pyridoxine hydrochloride, in powder form: wherein the spray flow rate of the mixture comprising from 30 to 45% of one or more coating agents, particularly 40% of (dewaxed) shellac in ethanol, is from 385 to 850 mg/min per Kg of inert nucleus; the solid addition rate of the powder is from 770 to 1700 mg /min per Kg of inert nucleus; and the relation between the spray flow rate of the mixture comprising the coating agents and the powdered mixture is from 25:75 to 40:60.

In an embodiment, the process of the invention further comprises a previous step of coating separately the inert nucleus with a simultaneous spraying of a mixture comprising from 30 to 45% by weight of one or more coating agents and adding the therapeutically effective amount of pyridoxine or a pharmaceutically acceptable salt thereof, particularly pyridoxine hydrochloride, in powder form: wherein the spray flow rate of the mixture comprising from 30 to 45% of one or more coating agents is from 385 to 850 mg/min per Kg of inert nucleus; the solid addition rate of the powder is from 770 to 1700 mg/min per Kg of inert nucleus; and the relation between the spray flow rate of the mixture comprising the coating agents and the solid addition rate of powdered mixture is from 25:75 to 40:60; and the coating agent is selected from the group consisting of microcrystalline cellulose, hydroxypropyl methyl cellulose (HPMC), polyvinyl pyrrolidone (PVP), shellac, polyethylene glycol (PEG) 6000, guar gum and starch; particularly (dewaxed) shellac.

In an embodiment, the process of the invention further comprises a previous step of coating separately the inert nucleus with a simultaneous spraying of a mixture comprising from 30 to 45% by weight of one or more coating agents and adding the therapeutically effective amount of pyridoxine or a pharmaceutically acceptable salt thereof; particularly pyridoxine hydrochloride, in power form: wherein the spray flow rate of the mixture comprising from 30 to 45% by weight of one or more coating agents is from 385 to 850 mg/min per Kg of inert nucleus; the solid addition rate of the powder is from 770 to 1700 mg/min per Kg of inert nucleus; and the relation between the spray flow rate of the mixture comprising the coating agents and the solid addition rate of powdered mixture is from 25:75 to 40:60; and the weight ratio between the therapeutically effective amount of pyridoxine or a pharmaceutically acceptable salt thereof; particularly pyridoxine hydrochloride in powder form and the mixture comprising the coating agents is from 25:75 to 40:60; particularly a weight ratio of 1:2.

In an embodiment, the process of the invention further comprises a previous step of coating separately the inert nucleus with a simultaneous spraying of a mixture comprising from 30 to 45% by weight of one or more coating agents and adding the therapeutically effective amount of pyridoxine or a pharmaceutically acceptable salt thereof; particularly pyridoxine hydrochloride in power form; wherein the spray flow rate of the mixture comprising from 30 to 45% by weight of one or more coating agents is from 385 to 850 mg/min per Kg of inert nucleus; the solid addition rate of the powder is from 770 to 1700 mg/min per Kg of inert nucleus; and the relation between the spray flow rate of the mixture comprising the coating agents and the solid addition rate of the powdered mixture is from 25:75 to 40:60 and pyridoxine or a pharmaceutically acceptable salt thereof; particularly pyridoxine hydrochloride, has a particle size equal to or lowers than 100 µm.

In an embodiment, the process of the invention further comprises a previous step of coating separately the inert nucleus with a simultaneous spraying of a mixture comprising from 30 to 45% by weight of one or more coating agents and adding the therapeutically effective amount of pyridoxine or a pharmaceutically acceptable salt thereof is one wherein the mixture comprising one or more coating agents is a solution further comprising one or more organic solvents; particularly in an amount of solvents from 65 to 83 g/kg of pellet, more particularly 71 g/kg pellets, having the mixture an overall solvent concentration from 55 to 70% by weigh, more particularly a 60% by weight of solvents. In an embodiment, the process is one wherein the mixture comprising the coating agents is a solution further comprising one or more organic solvents as defined above.

In an embodiment, the process of the invention further comprises an additional step which comprises coating the pellets of doxylamine or a pharmaceutically acceptable salt thereof; particularly doxylamine succinate, having the inner active coating layer by simultaneous spraying of a mixture comprising from 5 to 15% by weight of one or more enteric coating agents and adding from 5,5 to 6,0 g per kg of inert nucleus of the pore-forming agents in powder form wherein the spray flow rate of the mixture comprising from 5 to 15% by weight of one or more enteric coating agents is from 445 to 1000 mg/min per Kg of inert nucleus; the solid addition rate of the pore forming agent is from 45 to 100 mg/min per Kg of inert nucleus; and the relation between the spray flow rate of the mixture comprising the coating agents and the solid addition rate is from 87:13 to 93:7.

In an embodiment, the process of the invention further comprises an additional step which comprises coating the pellets of doxylamine or a pharmaceutically acceptable salt thereof, particularly doxylamine succinate, having the inner active coating layer by simultaneous spraying of a mixture comprising from 5 to 15% of the enteric coating agents and adding from 5,5 to 6,0 g per kg of inert nucleus of the pore-forming agents in powder form; wherein the spray flow rate of the mixture comprising from 5 to 15% by weight of one or more enteric coating agents is from 445 to 1000 mg/min per kg of inert nucleus; the solid addition rate of the pore forming agent is from 45 to 100 mg/min per Kg of inert nucleus; and the relation between the spray flow rate of the mixture comprising the coating agents and the solid addition rate is from 87:13 to 93:7 and the enteric coating agents are as defined above, particularly is methacrylic acid-methyl methacrylate copolymer (Eudragit L).

In an embodiment, the process of the invention further comprises an additional step which comprises coating the pellets of doxylamine or a pharmaceutically acceptable salt thereof, particularly doxylamine succinate, having the inner active release coating layer by simultaneous spraying of a mixture comprising from 5 to 15% by weight of the enteric coating agents and adding from 5,5 to 6,0 g per Kg of inert nucleus of the pore-forming agents in powder form; wherein the spray flow rate of the mixture comprising from 5 to 15% of one or more enteric coating agents is from 445 to 1000 mg/min per kg of inert nucleus, particularly 910 mg/min per kg of inert nucleus; the solid addition rate of the pore forming agent is from 45 to 100 mg/min per kg of inert nucleus; and the relation between the spray flow of the mixture comprising the coating agents and the powdered mixture is from 87:13 to 93:7, particularly 90:10; and the pore-forming agent is as defined above, particularly talc.

In an embodiment, the process of the invention further comprises an additional step which comprises coating the pellets of doxylamine or a pharmaceutically acceptable salt thereof, particularly doxylamine succinate, having the inner active release coating layer by simultaneous spraying of a mixture comprising from 5 to 15% by weight of the enteric coating agents and adding from 5.5 to 6.0 g per kg of inert nucleus, particularly 5.7 g per kg of inert nucleus, of the pore-forming agents in powder form, wherein the mixture comprising from 5 to 15% by weight of the enteric coating agents is a mixture further comprising one or more organic solvents; particularly in an overall amount of solvents from 48 to 55 g per kg of inert nucleus, more particularly 51 g per kg of inert nucleus, having the mixture an overall solvents concentration of 85 to 95% by weight, more particularly in a 90% by weight of solvents. In an embodiment, the process is one wherein the mixture comprising the coating agents is a solution further comprising one or more organic solvents as defined above. In an embodiment, the process of the present invention which comprises preparing the first plurality of modified release pellets of doxylamine or a pharmaceutically acceptable salt thereof by:
- coating the pellets of doxylamine or a pharmaceutically acceptable salt thereof having the inner active coating layer and the intermediate enteric coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40.

In an embodiment, the process of the present invention which comprises preparing the first plurality of modified release pellets of doxylamine or a pharmaceutically acceptable salt thereof by:
- coating the pellets of doxylamine or a pharmaceutically acceptable salt thereof having the inner active release coating layer by simultaneous spraying of a mixture comprising from 5 to 15% of the enteric coating agents and adding from 5,5 to 6,0 g per kg of inert nucleus of the pore-forming agents in powder form; wherein the spray flow rate of the mixture comprising from 5 to 15% by weight of one or more enteric coating agents is from 445 to 1000 mg/min per kg of inert nucleus; the solid addition rate of the pore forming agent is from 45 to 100 mg/min per Kg of inert nucleus; and the relation between the spray flow rate of the mixture comprising the coating agents and the solid addition rate is from 87:13 to 93:7;
   and
- coating the pellets of doxylamine or a pharmaceutically acceptable salt thereof having the inner active coating layer and the intermediate enteric coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg/min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40.

In an embodiment, the process of the present invention which comprises preparing the first plurality of modified release pellets of doxylamine or a pharmaceutically acceptable salt thereof by:
- coating separately the inert nucleus with a simultaneous spraying of a mixture comprising from 30 to 40% by weight one or more coating agents and adding a powdered mixture of the therapeutically effective amount of doxylamine or a pharmaceutically acceptable salt thereof, from 22 to 30% by weight of the pore-forming agents, and optionally one or more pharmaceutically acceptable excipients; wherein the spray flow rate of the mixture comprising from 30 to 40% by weight one or more coating agents is from 445 to 1000 mg/min per Kg of inert nucleus; the solid addition rate of the powdered mixture is from 1,64 to 3,62 g/min per Kg of inert nucleus; and the relation between the spray flow rate of the mixture comprising the coating agents and the solid addition rate of the powdered mixture is from 15:85 to 25:75;
- coating the pellets of doxylamine or a pharmaceutically acceptable salt thereof having the inner active coating layer by simultaneous spraying of a mixture comprising from 5 to 15% of the enteric coating agents and adding from 5,5 to 6,0 g per kg of inert nucleus of the pore-forming agents in powder form; wherein the spray flow rate of the mixture comprising from 5 to 15% by weight of one or more enteric coating agents is from 445 to 1000 mg/min per kg of inert nucleus; the solid addition rate of the pore forming agent is from 45 to 100 mg/min per Kg of inert nucleus; and the relation between the spray flow rate of the mixture comprising the coating agents and the solid addition rate is from 87:13 to 93:7;
   and
- coating the pellets of doxylamine or a pharmaceutically acceptable salt thereof having the inner active coating layer and the intermediate enteric coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40.

In an embodiment, the process of the present invention which comprises preparing the second plurality of modified release pellets of pyridoxine or a pharmaceutically acceptable salt thereof by:
- coating the pellets of pyridoxine or a pharmaceutically acceptable salt thereof; particularly doxylamine succinate, having the inner active coating layer by simultaneous spraying simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40.

In an embodiment, the process of the present invention which comprises preparing the second plurality of modified release pellets of pyridoxine or a pharmaceutically acceptable salt thereof by:
- coating separately the inert nucleus with a simultaneous spraying of a mixture comprising from 30 to 45% of one or more coating agents and adding the therapeutically effective amount of pyridoxine or a pharmaceutically acceptable salt thereof in powder form: wherein the spray flow rate of the mixture comprising from 30 to 45% of one or more coating agents is from 385 to 850 mg/min per Kg of inert nucleus; the solid addition rate of the powder is from 770 to 1700 mg /min per Kg of inert nucleus; and the relation between the spray flow rate of the mixture comprising the coating agents and the powdered mixture is from 25:75 to 40:60; and
- coating the pellets of pyridoxine or a pharmaceutically acceptable salt thereof; particularly doxylamine succinate, having the inner active coating layer by simultaneous spraying simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg / min per kg of inert nucleus; the solid addition rate of the pore-forming agents is from 75 to 500 mg/min per Kg of inert nucleus, particularly from 85 to 425 mg/min per Kg of inert nucleus; and the relation between the spray flow rate of the mixture comprising the coating agents and the addition rate of the pore-forming agents in solid form is from 90:10 to 60:40.

In an embodiment, each one of the coating steps are performed at a temperature from 10°C to 30°C; particularly from 15°C to 25°C; more particularly from 17°C to 22°C.

In an embodiment, each one of the coating steps are performed at an airflow from 0 a 20 m³/h per Kg of inert nucleus; particularly from 0 to 4 m3 /h per Kg of inert nucleus. The airflow is controlled using an anemometer type air inlet detection system installed in the duct between the fan and the coating pan, or using another equivalent system.

In an embodiment, the process of the present invention further comprises one or more additional steps of drying separately each one of the plurality of pellets obtained in each one of the coating step in order to remove the solvents and humidity; and optionally another further step of sieving the plurality of pellets if agglomeration of powder into granules is observed. In an embodiment, the drying steps are performed at a temperature from 20°C to 60°C; preferably from 25°C to 45°C.

In a particular embodiment, the inter-coating drying steps are carried out during not less than 1 h at a temperature from 15°C to 45 °C, with a coating pan speed from 0 to 10 rpm and with an airflow higher than 1 m³/h per kg of inert nucleus.

In a particular embodiment, the drying step of the external coating of the pellets is carried out during not less than 8 h and not more than 12 h at a temperature from 15 °C to 60 °C, preferably from 25°C to 50 °C; more particularly from 40°C to 45 °C, with coating pan speed from 0 to 10 rpm and with an airflow higher than 1 m³/ h per kg of inert nucleus.

In an embodiment, the process of the present invention further comprises one or more additional steps of sieving the plurality of pellets until having a sieving size from equal to or higher than 500 µm to equal to or lower than 1500 µm; particularly equal to or higher than 600 µm to equal to or lower than 1320 µm; more particularly from equal to or higher than 710 µm to equal to or lower than 1180 µm.

In an embodiment, the process is one wherein the modified release multiple unit oral dosage form exhibits a dissolution profile according to which:
from 10% to 35% by weight of doxylamine content is dissolved at 1^{st}h in 0.1 N HCI medium (pH = 1);
   then, the medium is replaced by a pH = 4.5 medium (0.05 M acetate buffer) and at 4^{th} h from an accumulated 45% to 70% by weight of doxylamine initial content is dissolved;
   then, the medium is replaced by a pH = 6.8 medium (0.05 M phosphate buffer) and at 7^{th} h at least an accumulated 80% of doxylamine initial content is dissolved
from 10% to 35% by weight of pyridoxine content is dissolved at 1^{st}h in 0.1 N HCI medium (pH = 1);
   then, the medium is replaced by a pH = 4.5 medium (0.05 M acetate buffer) and at 4^{th} h from an accumulated 40% to 65% by weight of pyridoxine initial content is dissolved;
   then, the medium is replaced by a pH = 6.8 medium (0.05 M phosphate buffer) and at 7^{th} h at least an accumulated 80% of pyridoxine initial content is dissolved
wherein the dissolution profile is measured using a USP type II apparatus (basket), placing the composition in 900mL of the corresponding media / buffer at 37°C ±0.5 °C and 100 rpm.

In an embodiment, the process of the present invention wherein the modified release multiple unit oral dosage form comprises:
a first plurality of modified release pellets of doxylamine or a pharmaceutically acceptable salt thereof comprising:
   - an inert nucleus;
   - an inner active coating layer comprising a therapeutically effective amount of doxylamine or a pharmaceutically acceptable salt thereof, from 7 to 11 % by weight of one or more coating agents and from 20 to 28 % by weight of one or more pore-forming agent; and optionally one or more pharmaceutically acceptable excipients;
   - an intermediate enteric release coating layer comprising from 45 to 65 % by weight of one or more enteric coating agents and from 55 to 35 % by weight of one or more pore-forming agent; and
   - an external modified release coating layer comprising from 8 to 14 % by weight of one or more enteric coating agents, from 38 to 46 % by weight of one or more modified release coating agents and from 42 to 52 % by weight of one or more pore-forming agent;
      and
a second plurality of modified release pellets of pyridoxine or a pharmaceutically acceptable salt thereof comprising:
   - an inert nucleus;
   - an inner active coating layer comprising a therapeutically effective amount of pyridoxine or a pharmaceutically acceptable salt thereof, and from 14 to 20 % by weight of one or more coating agents; and
   - an external modified release coating layer comprising from 2 to 6 % by weight of one or more enteric coating agents, from 30 to 45 % by weight of one or more modified release coating agents and from 50 to 65 % by weight of one or more pore-forming agents.

In an embodiment, the process of the invention further comprises an additional step of encapsulating the first plurality of pellets of doxylamine or a pharmaceutically acceptable salt thereof and the second plurality of pellets of pyridoxine or a pharmaceutically acceptable salt thereof into capsules; particularly into hard capsules.

The encapsulating step can be carried out by methods known in the art.

### Examples

### 1. Process for manufacturing capsules of pellets of doxylamine succinate and pyridoxine hydrochloride of the present invention

### 1.1. Quantitative composition per hard capsule

The amount of each one of the ingredients per capsule is as follows:

| **Ingredient** | **Quantity per capsule (mg)** | **Function** |
|---|---|---|
| **Active ingredients** | | |
| Doxylamine succinate | 10.0 | Active ingredient |
| Pyridoxine hydrochloride | 10.0 | Active ingredient |

| **Excipients** | | |
|---|---|---|
| Sugar spheres | 79.5 | Inert nucleus |
| (dewaxed) Shellac | 7.6 | Coating agent |
| Polyvinyl pyrrol idone (Povidone K-30) | 0.2 | Coating agent |
| Talc | 9.5 | Pore-forming agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard gelatine capsule⁽¹⁾ | 48 | Packing of dose unit |
| Capsule fill weight | 118.6 | |
| Capsule total weight | 166.6 | |

| | | |
|---|---|---|
| (1) The gelatine capsule is made of 0.0211% of indigotine-FD&C blue 2 (E132), 0.01% of quinoline yellow (E104), 1.8% of titanium dioxide (E171) and q.s.p. 100% of gelatine. | | |

### 1.2. Quantitative composition per plurality of pellets

### 1.2.1. Composition of the pellets of doxylamine succinate

The amount of each one of the ingredients per a batch of about 180 Kg of the pellets of doxylamine succinate is as follows:

| **Layer of the pellet** | **Ingredients** | **function** | **Amount per batch (kg)** |
|---|---|---|---|
| Core | Sugar spheres of sucrose and starch | Inert nucleus | 112.564 |
| Inner active coating layer | Doxylamine succinate | Active ingredient | 30.200 |
| | Talc | Pore-forming agent | 10.067 |
| | Silica colloidal anhydrous (Aerosil 200 pharma) | Anti-caking agent, gliding agent | 2.013 |
| | Polyvinyl pyrrolidone 20% solution in ethanol (Povidone K-30) | Coating agent solution | 3.020 |
| | (dewaxed) Shellac 40% solution in ethanol | Coating agent solution | 8.419 |
| Intermediate enteric release layer | Talc | Pore-forming agent | 0.641 |
| | Methacrylic acid and methyl methacrylate copolymer (1:1) (Eudragit L 10% in acetone | Enteric release coating agent mixture | 6.406 |
| External modified release layer | Methacrylic acid and methyl methacrylate copolymer (1:1) (Eudragit L) 10% in acetone | Enteric release coating agent mixture | 21.155 |
| | (dewaxed) Shellac 40% solution in ethanol | Modified release coating agent solution | 21.156 |
| | Talc | Pore-forming agent | 9.425 |
| **Total dry weight: 180.14** | | | |

### 1.2.2. Composition of the pellets of pyridoxine hydrochloride

The amount of each one of the ingredients per a batch of about 180 Kg of the pellets of pyridoxine hydrochloride is as follows:

| **Layer of the pellets** | **Ingredients** | **function** | **Amount per batch (Kg)** |
|---|---|---|---|
| Core | Sugar spheres of sucrose and starch | Inert nucleus | 129.285 |
| Inner active coating layer | pyridoxine hydrochloride | Active ingredient | 30.600 |
| | (dewaxed) Shellac 40% solution in ethanol | Coating agent solution | 15.300 |
| External modified release layer | Methacrylic acid and methyl methacrylate copolymer (1:1) (Eudragit L) 10% in acetone | Enteric release coating agent mixture | 4.896 |
| | (dewaxed) Shellac 40% solution in ethanol | Modified release coating agent solution | 12.677 |
| | Talc | Pore-forming agent | 8.743 |
| **Total dry weight:180.31** | | | |

### 1.3. Preparation process by powder coating

### 1.3.1. Population of pellets of doxylamine succinate

### A. Preparation of phases

Phase 1-powdered mixture for the preparation of the inner active coating layer: The above-mentioned amount of doxylamine succinate, aerosil 200 pharma and talc disclosed above were mixed in a coating pan.

Phase 2-binding solution for the preparation of the inner active coating layer: The above-mentioned amount of povidone K30 20 % in ethanol and (dewaxed) shellac 40 % in ethanol were mixed.
Phase 3-coating solution for the preparation of the external coating: The above-mentioned amount of (dewaxed) shellac 40% in ethanol and Eudragit L 10% in acetone were mixed.

### B. Preparation process

### -inner active coating layer

The sugar spheres were transferred into a coating pan, and then binding solution phase 2 was sprayed over the sugar spheres, kept at in rotation in the coating pan, at a spray flow rate of 100 g/min. While phase 2 was sprayed, powdered mixture Phase 1 was added (in solid form) over the sugar spheres at a solid addition rate of 370 g/min. The spraying step was performed at a rotation rate of 16 rpm, keeping the core temperature of the pellets between 17°C and 22°C and the airflow lower than 100 m³/h.

The coated pellets thus obtained were dried during not less than 2 h at room temperature by keeping them rotate in the coating pan at a rate comprised between 0 and 10 rpm and with an airflow >130 m³/h to obtain the dried coated pellets with the inner active coating layer having the active ingredient. If necessary, the dried pellets can be sieved and unwanted particle size, powders and aggregates were discarded.

### -intermediate enteric release coating layer

On the coated active pellets obtained in previous step, a mixture of Eudragit L 10 % in acetone was sprayed at a spray flow rate of 100 g/min per kg of inert nucleus. While Eudragit was sprayed, talc in solid form was applied over the pellets to obtain the pellets coated with the inner active coating layer and the intermediate enteric release coating layer at a solid addition rate of 10 g/min and at a rotation rate of 16 rpm, keeping the core temperature of the pellets between 17°C and 22°C and the airflow lower than 100 m³/h. The obtained coated pellets were dried during not less than 1 h at room temperature by keeping them rotate in the coating pan at a rate comprised between 0 and 10 rpm and with an airflow >130 m³/h.

### -external modified release coating layer

Then, phase 3 was sprayed over the bi-layered coated pellets obtained in previous step at a spray flow rate of 100 g/min. While phase 3 was sprayed, talc was applied in solid form at a solid addition rate of 20 g/min and at a rotation rate of 16 rpm, keeping the core temperature of the pellets between 17°C and 22°C and the airflow lower than 100 m³/h. The obtained coated pellets were dried during not less than 8 h and up to 12 h at 40-45 °C by keeping them rotate in the coating pan at a rate comprised between 0 and 10 rpm and with an airflow >130 m³/h

The pellet thus obtained has the target dissolution profile. If necessary, the dried pellets can be sieved and unwanted particle size, powders and aggregates were discarded.

The modified release pellets of doxylamine succinate thus obtained were stored in 50 kg closed double food pharmaceutical grade polyethylene bags inside closed High Density Polyethylene drums.

### 1.3.2. Plurality of pellets of pyridoxine hydrochloride

### A. Preparation of phases

Phase 4 -coating solution for the preparation of the external coating: The above-mentioned amount of (dewaxed) shellac 40% in ethanol and Eudragit L 10% in acetone were mixed.

### B. Preparation process

### -inner active coating layer

The sugar spheres were transferred into a coating pan, and then a solution of (dewaxed) shellac 40 % in ethanol was sprayed over the sugar spheres kept in rotation at a rotation speed of 16 rpms and at a spray flow rate of 100 g/min in the coating pan. While a solution of (dewaxed) shellac 40 % in ethanol was sprayed, then pyridoxine hydrochloride was added in solid form over the sugar spheres at a solid addition rate of 200 g min, keeping the core temperature of the pellets between 17°C and 22°C and the airflow lower than 100 m³/h

The coated pellets thus obtained were dried during not less than 2 h at room temperature by keeping them rotate in the coating pan at a rate comprised between 0 and 10 rpm and with an airflow >130 m³/h to obtain the dried coated pellets with the inner active coating layer having the active ingredient. If necessary, the dried pellets can be sieved and unwanted particle size, powders and aggregates were discarded.

### -external modified release coating layer

On the coated active pellets obtained in previous step, phase 4 was sprayed at flow rate of 100 g/min. While solution phase 4 was sprayed, talc was applied in solid form at a solid addition rate of 50 g/min and a rotation rate of 16 rpm, keeping the core temperature of the pellets between 17°C and 22°C and the airflow lower than 100 m³/h. The obtained coated pellets were dried during not less than 8 h and up to 12 h at 40-45 °C by keeping them rotate in the coating pan at a rate comprised between 0 and 10 rpm and with an airflow >130 m³/h.

The pellet thus obtained has the target dissolution profile. If necessary, the dried pellets can be sieved and unwanted particle size, powders and aggregates were discarded.

The modified release pellets of pyridoxine hydrochloride thus obtained were stored in 50 kg closed double food pharmaceutical grade polyethylene bags inside closed High Density Polyethylene drums.

### Capsule filling

Each Hard capsule was filled with 60 mg of the modified release pellets of doxylamine succinate and 60 mg of the modified release pellets of pyridoxine hydrochloride of the present invention as defined above using a Bosch Zanassi E48 automatic capsule-filling machine.

### 2. Dissolution Test

### Dissolution profile

The target dissolution profile requires that both the doxylamine succinate and the pyridoxine hydrochloride were slightly dissolved under the stomach conditions and that the major therapeutic concentration was achieved in the intestinal tract due to its rapid dissolution rate. In particular, the process for the preparation of the present invention allows obtaining capsules, filled with such a modified release pellets of doxylamine succinate and modified release pellets of pyridoxine hydrochloride, which exhibit a dissolution profile according to which:
from 10% to 35% by weight of doxylamine content is dissolved at 1^{st}h in 0.1 N HCI medium (pH = 1);
   then, the medium is replaced by a pH = 4.5 medium (0.05 M acetate buffer) and at 4^{th} h from an accumulated 45% to 70% by weight of doxylamine initial content is dissolved;
   then, the medium is replaced by a pH = 6.8 medium (0.05 M phosphate buffer) and at 7^{th} h at least an accumulated 80% of doxylamine initial content is dissolved
from 10% to 35% by weight of pyridoxine content is dissolved at 1^{st}h in 0.1 N HCI medium (pH = 1);
   then, the medium is replaced by a pH = 4.5 medium (0.05 M acetate buffer) and at 4^{th} h from an accumulated 40% to 65% by weight of pyridoxine initial content is dissolved;
   then, the medium is replaced by a pH = 6.8 medium (0.05 M phosphate buffer) and at 7^{th} h at least an accumulated 80% of pyridoxine initial content is dissolved.
wherein the dissolution profile is measured using a USP type II apparatus (basket), placing the composition in 900mL of the corresponding media / buffered 37°C ±0.5 °C and 100 rpm.

### Conditions of the dissolution bath

- Paddle speed: 100 rpm
- Temperature of dissolution medium: 37 °C ± 0.5 °C
- Dissolution media: hydrochloric acid 0.1 N
- Vessel volume: 900 mL
- Time: 1 hour
- Dissolution media: pH 4.5; 0.05 M acetate buffer
- Vessel volume: 900 mL
- Time: From the 1^{st} h to the 4^{th} hour
- Dissolution media: pH 6.8; 0.05 M phosphate buffer
- Vessel volume: 900 mL
- Time: From the 4^{th} h to the 7^{th} hour

### Conditions of the chromatographic analysis

- Sample preparation: Take an aliquot of approximately 10 ml and filter it through 0.70 µm membrane filter, then filter it through another 0.22 µm membrane filter.
- Flux: 1 mL/min
- Column: Kromasil 100-5 C18, 150 x 4.0 mm
- Phases: methanol in water
- Injection volume: 100 µL
- Excitation wavelength: 220 nm
- Chromatographic time: 25 min.
- Aqueous phase: Ammonium acetate buffer 0.06 M pH 5.0 + 0,1% sodium hexanesulfonate (PICB6)):
- Gradient:

| Time (min) | Methanol (%) | Aqueous phase (%) |
|---|---|---|
| 0 | 20 | 80 |
| 4 | 32.5 | 80 |
| 8 | 100 | 50 |
| 13 | 100 | 50 |
| 17 | 20 | 80 |
| 25 | 20 | 80 |

### Results

The dissolution profile of the capsule of the present invention which comprises pellets of doxylamine succinate and pyridoxine hydrochloride

| pH | Time (hours) | Dissolved doxylamine succinate (%) | Dissolved pyridoxine hydrochloride (%) |
|---|---|---|---|
| 1 | 0.5 | 11.3 | 9.3 |
| | **1** | **22.3** | **16.5** |
| 4.5 | 2 | 38.7 | 33.0 |
| | 3 | 47.6 | 44.0 |
| | **4** | **54.9** | **52.8** |
| 6.8 | 5 | 84.6 | 74.4 |
| | 6 | 93.8 | 90.2 |
| | **7** | **97.2** | **97.8** |

The dissolution profile as disclosed above show that the capsules of the invention have the required dissolution profile. Thus, the dissolution of both doxylamine succinate and pyridoxine hydrochloride when it is submitted to stomach conditions is, at least a 10% of the total amount in 1 hour and at least a 40% of doxylamine succinate and pyridoxine hydrochloride after 4 hours is dissolved when it is submitted to duodenal conditions (pH = 4.5) and at least an 80% of doxylamine succinate and pyridoxine hydrochloride after 7 hours is dissolved when it is submitted to colon conditions.

## Claims

1. A process for the preparation of a modified release multiple unit oral dosage forms comprising:
a first plurality of modified release pellets of doxylamine or a pharmaceutically acceptable salt thereof comprising:
- an inert nucleus;
- an inner active coating layer comprising a therapeutically effective amount of doxylamine or a pharmaceutically acceptable salt thereof, one or more coating agents and one or more pore-forming agent; and optionally one or more pharmaceutically acceptable excipients;
- an intermediate enteric release coating layer comprising one or more enteric coating agents and one or more pore-forming agent; and
- an external modified release coating layer comprising one or more enteric coating agents, one or more modified release coating agents and one or more pore-forming agent;
and
a second plurality of modified release pellets of pyridoxine or a pharmaceutically acceptable salt thereof comprising:
- an inert nucleus;
- an inner active coating layer comprising a therapeutically effective amount of pyridoxine or a pharmaceutically acceptable salt thereof, and one or more coating agents; and
- an external modified release coating layer comprising one or more enteric coating agents, one or more modified release coating agents and one or more pore-forming agents;
wherein the process comprises:
- preparing the first plurality of modified release pellets of doxylamine or a pharmaceutically acceptable salt thereof by coating the pellets of doxylamine or a pharmaceutically acceptable salt thereof having the inner active coating layer and the intermediate enteric coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg/min per kg of inert nucleus; the solid addition rate of the pore-forming agents is from 75 to 500 mg/min per Kg of inert nucleus; and the relation between the spray flow rate of the mixture comprising the coating agents and the addition rate of the pore-forming agents in solid form is from 90:10 to 60:40; and
- preparing the second plurality of modified release pellets of pyridoxine or a pharmaceutically acceptable salt thereof by coating the pellets of pyridoxine or a pharmaceutically acceptable salt thereof having the inner active coating layer by simultaneous spraying a mixture comprising from 2.0 to 7.5 % by weight of the enteric coating agents and from 15.0 to 35.0 % by weight of the modified release coating agents in a weight ratio from 5:95 to 30:70; and adding the pore-forming agents in powder form, wherein: the spray flow rate of the mixture comprising the coating agents is from 300 to 1200 mg/min per kg of inert nucleus; the solid addition rate of the pore-forming agents is from 75 to 500 mg/min per Kg of inert nucleus; and the relation between the spray flow rate of the mixture comprising the coating agents and the addition rate of the pore-forming agents in solid form is from 90:10 to 60:40.

2. The process according to claim 1, wherein the process comprises:
- preparing the first plurality of modified release pellets of doxylamine or a pharmaceutically acceptable salt thereof as defined in claim 1, wherein the solid addition rate of the pore-forming agents is from 88 to 195 mg/min per Kg of inert nucleus;
and
- preparing the second plurality of modified release pellets of pyridoxine or a pharmaceutically acceptable salt thereof as defined in claim 1, wherein the solid addition rate of the pore-forming agents is from 193 to 425 mg/min per Kg of inert nucleus

3. The process according to any of the claims 1 or 2, wherein the process comprises:
- preparing the first plurality of modified release pellets of doxylamine or a pharmaceutically acceptable salt thereof as defined in claim 1, wherein the relation between the spray flow rate of the mixture comprising the coating agents and the solid addition rate of the pore-forming agents is from 90:10 to 60:40;
and
- preparing the second plurality of modified release pellets of pyridoxine or a pharmaceutically acceptable salt thereof as defined in claim 1, wherein the relation between the spray flow rate of the mixture comprising the coating agents and the solid addition rate of the pore-forming agents is from 90:10 to 60:40.

4. The process according to any of the claims 1-3, wherein the mixture comprises from 2,5 to 5.5% by weight of the enteric coating agents and from 18 to 30% by weight of the modified release coating agents.

5. The process according to any of the claims 1-4, wherein the process further comprises a previous step of coating separately:
- coating the pellets of doxylamine or a pharmaceutically acceptable salt thereof having the inner active coating layer by simultaneous spraying of a mixture comprising from 5 to 15% of the enteric coating agents and adding from 5.5 to 6.0 g per kg of inert nucleus of the pore-forming agents in powder form; wherein the spray flow rate of the mixture comprising from 5 to 15% by weight of one or more enteric coating agents is from 445 to 1000 mg/min per kg of inert nucleus; the solid addition rate of the pore forming agent is from 45 to 100 mg/min per Kg of inert nucleus; and the relation between the spray flow rate of the mixture comprising the coating agents and the solid addition rate is from 87:13 to 93:7;
and
- coating the inert nucleus with a simultaneous spraying of a mixture comprising from 30 to 45% of one or more coating agents and adding the therapeutically effective amount of pyridoxine or a pharmaceutically acceptable salt thereof in powder form: wherein the spray flow rate of the mixture comprising from 30 to 45% of one or more coating agents is from 385 to 850 mg/min per Kg of inert nucleus; the solid addition rate of the powder is from 770 to 1700 mg/min per Kg of inert nucleus; and the relation between the spray flow rate of the mixture comprising the coating agents and the powdered mixture is from 25:75 to 40:60.

6. The process according to any of the claims 1-5, wherein the particle size of doxylamine or a pharmaceutically acceptable salt thereof and pyridoxine or a pharmaceutically acceptable salt thereof is from 1 µm to 250 µm.

7. The process according to any of the claims 1-6, wherein the process further comprises an additional step which comprises coating the inert nucleus with a simultaneous spraying of a mixture comprising from 30 to 40% by weight one or more coating agents and adding a powdered mixture of the therapeutically effective amount of doxylamine or a pharmaceutically acceptable salt thereof from 22 to 30% by weight of the pore-forming agents, and optionally one or more pharmaceutically acceptable excipients; wherein the spray flow rate of the mixture comprising from 30 to 40% by weight one or more coating agents is from 445 to 1000 mg/min per Kg of inert nucleus; the solid addition rate of the powdered mixture is from 1,64 to 3,62 g/min per Kg of inert nucleus; and the relation between the spray flow rate of the mixture comprising the coating agents and the solid addition rate of the powdered mixture is from 15:85 to 25:75.

8. The process according to any of the claims 1-7, wherein the pore-forming agent has a particle size is from 1 µm to 250 µm.

9. The process according to any of the claims 1-8, wherein the modified release multiple unit oral dosage form comprising doxylamine succinate and pyridoxine hydrochloride.

10. The process according to any of the claims 1-9, wherein the modified release multiple units oral dosage form is a hard capsule comprising from 5 mg to 50 mg per capsule of doxylamine succinate and from 5 mg to 50 mg per capsule of pyridoxine hydrochloride.

11. The process according to any of the claims 1-10, wherein the coating agents of the inner active coating layer of pellets of doxylamine or a pharmaceutically acceptable salt thereof are selected from the group consisting polyvinylpyrrolidone, shellac, hypromellose, hydroxypropylcellulose, microcrystalline cellulose and a mixture thereof; or alternatively
the pore-forming agent is selected from the group consisting of talc, micronized sugar, sodium or potassium chloride and a mixture thereof.

12. The process according to any of the claims 1-11, wherein the enteric coating agents is selected from the group consisting of copolymer of methacrylic acid and methyl methacrylate, copolymer of methacrylic acid and methyl acrylate, cellulose acetate phthalate, hydroxypropyl methyl cellulose phthalate, polyvinyl acetate phthalate sodium alginate, cellulose acetate trimellitate and a mixture thereof.

13. The process according to any of the claims 1-12, wherein the process further comprises an additional step of drying separately each one of the plurality of pellets obtained in each one of the coating step, wherein the drying step is performed at a temperature from 15 °C to 60 °C and an airflow >1 m³ / (h per kg inert nucleus) during the appropriate period of time for having less than 5000 ppm of each of the solvents used in the process.

14. The process according to any of the claims 1-13, wherein the modified release multiple unit oral dosage form exhibits a dissolution profile according to which:
from 10% to 35% by weight of doxylamine content is dissolved at 1^{st}h in 0.1 N HCI medium (pH = 1);
then, the medium is replaced by a pH = 4.5 medium (0.05 M acetate buffer) and at 4^{th} h from an accumulated 45% to 70% by weight of doxylamine initial content is dissolved;
then, the medium is replaced by a pH = 6.8 medium (0.05 M phosphate buffer) and at 7^{th} h at least an accumulated 80% of doxylamine initial content is dissolved
from 10% to 35% by weight of pyridoxine content is dissolved at 1^{st}h in 0.1 N HCI medium (pH = 1);
then, the medium is replaced by a pH = 4.5 medium (0.05 M acetate buffer) and at 4^{th} h from an accumulated 40% to 65% by weight of pyridoxine initial content is dissolved;
then, the medium is replaced by a pH = 6.8 medium (0.05 M phosphate buffer) and at 7^{th} h at least an accumulated 80% of pyridoxine initial content is dissolved.
wherein the dissolution profile is measured using a USP type II apparatus (basket), placing the composition in 900mL of the corresponding media / buffered 37°C ± 0.5 °C and 100 rpm.

15. The process according to any of the claims 1-14, wherein the modified release multiple unit oral dosage form comprises:
a first plurality of modified release pellets of doxylamine or a pharmaceutically acceptable salt thereof comprising:
- an inert nucleus;
- an inner active coating layer comprising a therapeutically effective amount of doxylamine or a pharmaceutically acceptable salt thereof, from 7 to 11 % by weight of one or more coating agents and from 20 to 28 % by weight of one or more pore-forming agent; and optionally one or more pharmaceutically acceptable excipients;
- an intermediate enteric release coating layer comprising from 45 to 65 % by weight of one or more enteric coating agents and from 55 to 35 % by weight of one or more pore-forming agent; and
- an external modified release coating layer comprising from 8 to 14 % by weight of one or more enteric coating agents, from 38 to 46 % by weight of one or more modified release coating agents and from 42 to 52 % by weight of one or more pore-forming agent;
and
a second plurality of modified release pellets of pyridoxine or a pharmaceutically acceptable salt thereof comprising:
- an inert nucleus;
- an inner active coating layer comprising a therapeutically effective amount of pyridoxine or a pharmaceutically acceptable salt thereof, and from 14 to 20 % by weight of one or more coating agents; and
- an external modified release coating layer comprising from 2to 6 % by weight of one or more enteric coating agents, from 30 to 45 % by weight of one or more modified release coating agents and from 50 to 65 % by weight of one or more pore-forming agents.

## Patentansprüche

1. Ein Verfahren zur Herstellung von einer oralen Arzneiform aus mehrfachen Einheiten mit modifizierter Freisetzung umfassend:
eine erste Vielzahl von Pellets mit modifizierter Freisetzung aus Doxylamin oder einem pharmazeutisch akzeptablen Salz davon, umfassend:
- einen inerten Kern;
- eine innere aktive Beschichtung umfassend eine therapeutisch wirksame Menge an Doxylamin oder einem pharmazeutisch akzeptablen Salz davon, ein oder mehrere Beschichtungsmittel und ein oder mehrere porenbildende Mittel; und wahlweise einen oder mehrere pharmazeutisch akzeptable Hilfsstoffe;
- eine mittlere magensaftresistente Freisetzungsbeschichtung umfassend ein oder mehrere magensaftresistente Beschichtungsmittel und ein oder mehrere porenbildende Mittel; und
- eine äußere Beschichtung mit modifizierter Freisetzung umfassend ein oder mehrere magensaftresistente Beschichtungsmittel, ein oder mehrere Beschichtungsmittel mit modifizierter Freisetzung und ein oder mehrere porenbildende Mittel;
und
eine zweite Vielzahl von Pellets mit modifizierter Freisetzung aus Pyridoxin oder einem pharmazeutisch akzeptablen Salz davon, umfassend:
- einen inerten Kern;
- eine innere aktive Beschichtung umfassend eine therapeutisch wirksame Menge an Pyridoxin oder einem pharmazeutisch akzeptablen Salz davon, und ein oder mehrere Beschichtungsmittel; und
- eine äußere Beschichtung mit modifizierter Freisetzung umfassend ein oder mehrere magensaftresistente Beschichtungsmittel, ein oder mehrere Beschichtungsmittel mit modifizierter Freisetzung und ein oder mehrere porenbildende Mittel;
wobei das Verfahren folgendes umfasst:
- das Herstellen von der ersten Vielzahl von Pellets mit modifizierter Freisetzung aus Doxylamin oder einem pharmazeutisch akzeptablen Salz davon durch die Beschichtung der Pellets aus Doxylamin oder einem pharmazeutisch akzeptablen Salz davon, welche die innere aktive Beschichtung und die mittlere magensaftresistente Beschichtung hat, indem eine Mischung umfassend von 2,0 bis 7,5 Gew.-% der magensaftresistenten Beschichtungsmittel und von 15,0 bis 35,0 Gew.-% der Beschichtungsmittel modifizierter Freisetzung bei einem Gewichtsverhältnis von 5:95 bis 30:70 besprüht werden; und gleichzeitig die porenbildenden Mittel in Pulverform hinzugefügt werden, wobei: die Besprühungsflussrate der Mischung umfassend die Beschichtungsmittel von 300 bis 1200 mg / min pro kg des inerten Kerns reicht; die Feststoffzugaberate der porenbildenden Mittel von 75 bis 500 mg/min pro Kg des inerten Kerns reicht; und das Verhältnis von der Besprühungsflussrate der Mischung umfassend die Beschichtungsmittel zur Zugaberate der porenbildenden Mittel in fester Form von 90:10 bis 60:40 reicht; und
- das Herstellen von der zweiten Vielzahl von Pellets mit modifizierter Freisetzung aus Pyridoxin oder einem pharmazeutisch akzeptablen Salz davon durch die Beschichtung der Pellets aus Pyridoxin oder einem pharmazeutisch akzeptablen Salz davon, welche die innere aktive Beschichtung hat, indem eine Mischung umfassend von 2,0 bis 7,5 Gew.-% der magensaftresistenten Beschichtungsmittel und von 15,0 bis 35,0 Gew.-% der Beschichtungsmittel modifizierter Freisetzung bei einem Gewichtsverhältnis von 5:95 bis 30:70 besprüht werden; und gleichzeitig die porenbildenden Mittel in Pulverform hinzugefügt werden, wobei: die Besprühungsflussrate der Mischung umfassend die Beschichtungsmittel von 300 bis 1200 mg / min pro kg des inerten Kerns reicht; die Feststoffzugaberate der porenbildenden Mittel von 75 bis 500 mg/min pro Kg des inerten Kerns reicht; und das Verhältnis von der Besprühungsflussrate der Mischung umfassend die Beschichtungsmittel zur Zugaberate der porenbildenden Mittel in fester Form von 90:10 bis 60:40 reicht.

2. Das Verfahren nach Anspruch 1, wobei das Verfahren folgendes umfasst:
- das Herstellen von der ersten Vielzahl von Pellets mit modifizierter Freisetzung aus Doxylamin oder einem pharmazeutisch akzeptablen Salz davon wie in Anspruch 1 definiert, wobei die Feststoffzugaberate der porenbildenden Mittel von 88 bis 195 mg/min pro Kg des inerten Kerns reicht;
und
- das Herstellen von der zweiten Vielzahl von Pellets mit modifizierter Freisetzung aus Pyridoxin oder einem pharmazeutisch akzeptablen Salz davon wie in Anspruch 1 definiert, wobei die Feststoffzugaberate der porenbildenden Mittel von 193 bis 425 mg/min pro Kg des inerten Kerns reicht.

3. Das Verfahren nach einem der Ansprüche 1 oder 2, wobei das Verfahren folgendes umfasst:
- das Herstellen von der ersten Vielzahl von Pellets mit modifizierter Freisetzung aus Doxylamin oder einem pharmazeutisch akzeptablen Salz davon wie in Anspruch 1 definiert, wobei das Verhältnis von der Besprühungsflussrate der Mischung umfassend die Beschichtungsmittel zur Feststoffzugaberate der porenbildenden Mittel von 90:10 bis 60:40 reicht;
und
- das Herstellen von der zweiten Vielzahl von Pellets mit modifizierter Freisetzung aus Pyridoxin oder einem pharmazeutisch akzeptablen Salz davon wie in Anspruch 1 definiert, wobei das Verhältnis von der Besprühungsflussrate der Mischung umfassend die Beschichtungsmittel zur Feststoffzugaberate der porenbildenden Mittel von 90:10 bis 60:40 reicht.

4. Das Verfahren nach einem der Ansprüche 1-3, wobei die Mischung von 2,5 bis 5,5 Gew.-% der magensaftresistente Beschichtungsmittel und von 18 bis 30 Gew.-% der Beschichtungsmittel mit modifizierter Freisetzung umfasst.

5. Das Verfahren nach einem der Ansprüche 1-4, wobei das Verfahren weiterhin einen vorherigen Schritt umfasst, in dem folgende Beschichtungen getrennt durchgeführt werden:
- die Beschichtung der Pellets aus Doxylamin oder einem pharmazeutisch akzeptablen Salz davon, welche die innere aktive Beschichtung haben, indem eine Mischung umfassend von 5 bis 15% der magensaftresistenten Beschichtungsmittel besprüht wird und gleichzeitig von 5,5 bis 6,0 g pro kg des inerten Kerns der porenbildenden Mittel in Pulverform hinzugefügt wird; wobei die Besprühungsflussrate der Mischung umfassend von 5 bis 15 Gew.-% von einem oder mehreren magensaftresistenten Beschichtungsmitteln von 445 bis 1000 mg/min pro kg des inerten Kerns reicht; die Feststoffzugaberate des porenbildenden Mittels von 45 bis 100 mg/min pro Kg des inerten Kerns reicht; und das Verhältnis von der Besprühungsflussrate der Mischung umfassend die Beschichtungsmittel zur Feststoffzugaberate von 87:13 bis 93:7 reicht;
und
- die Beschichtung des inerten Kerns mit einer gleichzeitigen Besprühung von einer Mischung umfassend von 30 bis 45% von einem oder mehreren Beschichtungsmitteln und der Hinzufügung von der therapeutisch wirksamen Menge an Pyridoxin oder einem pharmazeutisch akzeptablen Salz davon in Pulverform: wobei die Besprühungsflussrate der Mischung umfassend von 30 bis 45% von einem oder mehreren Beschichtungsmitteln von 385 bis 850 mg/min pro Kg des inerten Kerns reicht; die Feststoffzugaberate des Pulvers von 770 bis 1700 mg/min pro Kg des inerten Kerns reicht; und das Verhältnis von der Besprühungsflussrate der Mischung umfassend die Beschichtungsmittel zur gepulverten Mischung von 25:75 bis 40:60 reicht.

6. Das Verfahren nach einem der Ansprüche 1-5, wobei die Partikelgröße von Doxylamin oder einem pharmazeutisch akzeptablen Salz davon und Pyridoxin oder einem pharmazeutisch akzeptablen Salz davon von 1 µm bis 250 µm reicht.

7. Das Verfahren nach einem der Ansprüche 1-6, wobei das Verfahren weiterhin einen zusätzlichen Schritt umfasst, welcher die Beschichtung des inerten Kerns mit einer Besprühung von einer Mischung umfassend von 30 bis 40 Gew.-% von einem oder mehreren Beschichtungsmitteln und gleichzeitig der Hinzufügung von einer gepulverten Mischung der therapeutisch wirksamen Menge an Doxylamin oder einem pharmazeutisch akzeptablen Salz davon von 22 bis 30 Gew.-% der porenbildenden Mittel und, wahlweise, einem oder mehreren pharmazeutisch akzeptablen Hilfsstoffen umfasst; wobei die Besprühungsflussrate der Mischung umfassend von 30 bis 40 Gew.-% von einem oder mehreren Beschichtungsmitteln von 445 bis 1000 mg/min pro Kg des inerten Kerns reicht; die Feststoffzugaberate der gepulverten Mischung von 1,64 bis 3,62 g/min pro Kg des inerten Kerns reicht; und das Verhältnis von der Besprühungsflussrate der Mischung umfassend die Beschichtungsmittel zur Feststoffzugaberate der gepulverten Mischung von 15:85 bis 25:75 reicht.

8. Das Verfahren nach einem der Ansprüche 1-7, wobei das porenbildende Mittel eine Partikelgröße reichend von 1 µm bis 250 µm hat.

9. Das Verfahren nach einem der Ansprüche 1-8, wobei die orale Arzneiform aus mehrfachen Einheiten mit modifizierter Freisetzung Doxylaminsuccinat und Pyridoxinhydrochlorid umfasst.

10. Das Verfahren nach einem der Ansprüche 1-9, wobei die orale Arzneiform aus mehrfachen Einheiten mit modifizierter Freisetzung eine harte Kapsel umfassend von 5 mg bis 50 mg Doxylaminsuccinat pro Kapsel und von 5 mg bis 50 mg Pyridoxinhydrochlorid pro Kapsel ist.

11. Das Verfahren nach einem der Ansprüche 1-10, wobei die Beschichtungsmittel der inneren aktiven Beschichtung von Pellets aus Doxylamin oder einem pharmazeutisch akzeptablen Salz davon ausgewählt aus der Gruppe bestehend aus Polyvinylpyrrolidon, Schellack, Hypromellose, Hydroxypropylcellulose, mikrokristalliner Cellulose und einer Mischung davon sind; oder, ersatzweise,
wobei das porenbildende Mittel ausgewählt aus der Gruppe bestehend aus Talk, mikronisiertem Zucker, Natrium- oder Kaliumchlorid und einer Mischung davon ist.

12. Das Verfahren nach einem der Ansprüche 1-11, wobei das magensaftresistente Beschichtungsmittel ausgewählt ist aus der Gruppe bestehend aus Copolymer von Methacrylsäure und Methylmethacrylat, Copolymer von Methacrylsäure und Methylacrylat, Celluloseacetatphthalat, Hydroxypropylmethylcellulosephthalat, Polyvinylacetatphthalat, Natriumalginat, Celluloseacetattrimellitat und einer Mischung davon.

13. Das Verfahren nach einem der Ansprüche 1-12, wobei das Verfahren weiterhin einen zusätzlichen Schritt umfasst, in dem jede einzelne aus der Vielzahl von Pellets, die in jedem einzelnen Beschichtungsschritt erhalten wird, getrennt getrocknet wird, wobei der Trocknungsschritt bei einer Temperatur von 15 °C bis 60 °C und einer Luftströmung >1 m³ / (h pro kg des inerten Kerns) während des geeigneten Zeitraums, so dass weniger als 5000 ppm von jedem von den Lösungsmitteln in dem Verfahren verwendet wird, durchgeführt wird.

14. Das Verfahren nach einem der Ansprüche 1-13, wobei die orale Arzneiform aus mehrfachen Einheiten mit modifizierter Freisetzung ein Lösungsprofil aufweist, gemäß welchem:
von 10 Gew.-% bis 35 Gew.-% des Doxylamingehalts in der 1. Stunde in einem 0,1 N HCl-Medium (pH-Wert = 1) gelöst ist;
dann wird das Medium durch ein Medium mit einem pH-Wert = 4,5 (0,05 M Acetatpuffer) ersetzt und in der 4. Stunde ein kumuliertes 45 Gew.-% bis 70 Gew.-% des anfänglichen Doxylamingehalts gelöst ist;
dann wird das Medium durch ein Medium mit einem pH-Wert = 6,8 (0,05 M Phosphatpuffer) ersetzt und in der 7. Stunde mindestens ein kumuliertes 80% des anfänglichen Doxylamingehalts gelöst ist;
von 10 Gew.-% bis 35 Gew.-% des Pyridoxingehalts in der 1. Stunde in einem 0,1 N HCl-Medium (pH-Wert = 1) gelöst ist;
dann wird das Medium durch ein Medium mit einem pH-Wert = 4,5 (0,05 M Acetatpuffer) ersetzt und in der 4. Stunde ein kumuliertes 40 Gew.-% bis 65 Gew.-% des anfänglichen Pyridoxingehalts gelöst ist;
dann wird das Medium durch ein Medium mit einem pH-Wert = 6,8 (0,05 M Phosphatpuffer) ersetzt und in der 7. Stunde mindestens ein kumuliertes 80% des anfänglichen Pyridoxingehalts gelöst ist;
wobei das Lösungsprofil unter Verwendung von einem USP-Typ II-Gerät (Basket) gemessen wird, wobei die Zusammensetzung in 900 mL der entsprechenden Medien / gepuffert bei 37 °C ± 0,5 °C und 100 rpm gesetzt wird.

15. Das Verfahren nach einem der Ansprüche 1-14, wobei die orale Arzneiform aus mehrfachen Einheiten mit modifizierter Freisetzung folgendes umfasst:
eine erste Vielzahl von Pellets mit modifizierter Freisetzung aus Doxylamin oder einem pharmazeutisch akzeptablen Salz davon, umfassend:
- einen inerten Kern;
- eine innere aktive Beschichtung umfassend eine therapeutisch wirksame Menge an Doxylamin oder einem pharmazeutisch akzeptablen Salz davon, von 7 bis 11 Gew.-% von einem oder mehreren Beschichtungsmitteln und von 20 bis 28 Gew.-% von einem oder mehreren porenbildenden Mitteln; und wahlweise einen oder mehrere pharmazeutisch akzeptable Hilfsstoffe;
- eine mittlere magensaftresistente Freisetzungsbeschichtung umfassend von 45 bis 65 Gew.-% von einem oder mehreren magensaftresistenten Beschichtungsmitteln und von 55 bis 35 Gew.-% von einem oder mehreren porenbildenden Mitteln; und
- eine äußere Beschichtung mit modifizierter Freisetzung umfassend von 8 bis 14 Gew.-% von einem oder mehreren magensaftresistenten Beschichtungsmitteln, von 38 bis 46 Gew.-% von einem oder mehreren Beschichtungsmitteln mit modifizierter Freisetzung und von 42 bis 52 Gew.-% von einem oder mehreren porenbildenden Mitteln;
und
eine zweite Vielzahl von Pellets mit modifizierter Freisetzung aus Pyridoxin oder einem pharmazeutisch akzeptablen Salz davon, umfassend:
- einen inerten Kern;
- eine innere aktive Beschichtung umfassend eine therapeutisch wirksame Menge an Pyridoxin oder einem pharmazeutisch akzeptablen Salz davon, und von 14 bis 20 Gew.-% von einem oder mehreren Beschichtungsmitteln; und
- eine äußere Beschichtung mit modifizierter Freisetzung umfassend von 2 bis 6 Gew.-% von einem oder mehreren magensaftresistenten Beschichtungsmitteln, von 30 bis 45 Gew.-% von einem oder mehreren Beschichtungsmitteln mit modifizierter Freisetzung und von 50 bis 65 Gew.-% von einem oder mehreren porenbildenden Mitteln.

## Revendications

1. Un procédé de préparation d'une formes galéniques orales à unités multiples à libération modifiée comprenant :
une première pluralité de pellets à libération modifiée de doxylamine ou un sel pharmaceutiquement acceptable de celle-ci comprenant :
- un noyau inerte :
- une couche de revêtement active intérieure comprenant une quantité thérapeutiquement efficace de doxylamine ou un sel pharmaceutiquement acceptable de celle-ci, un ou plusieurs agents de revêtement et un ou plusieurs agents de formation de pores ; et facultativement un ou plusieurs excipients pharmaceutiquement acceptables ;
- une couche de revêtement entérique moyenne comprenant un ou plusieurs agents de revêtement entérique et un ou plusieurs agents de formation de pores ; et
- une couche de revêtement à libération modifiée extérieure comprenant un ou plusieurs agents de revêtement entérique, un ou plusieurs agents de revêtement à libération modifiée et un ou plusieurs agents de formation de pores ;
et
une seconde pluralité de pellets à libération modifiée de pyridoxine ou un sel pharmaceutiquement acceptable de celle-ci comprenant :
- un noyau inerte :
- une couche de revêtement active intérieure comprenant une quantité thérapeutiquement efficace de pyridoxine ou un sel pharmaceutiquement acceptable de celle-ci, et un ou plusieurs agents de revêtement ; et
- une couche de revêtement à libération modifiée extérieure comprenant un ou plusieurs agents de revêtement entérique, un ou plusieurs agents de revêtement à libération modifiée et un ou plusieurs agents de formation de pores ;
où le procédé comprend :
- préparer la première pluralité de pellets à libération modifiée de doxylamine ou un sel pharmaceutiquement acceptable de celle-ci en revêtant les pellets de doxylamine ou d'un sel pharmaceutiquement acceptable de celle-ci ayant la couche de revêtement active intérieure et la couche de revêtement entérique moyenne en pulvérisant un mélange comprenant de 2,0 à 7,5 % en poids des agents de revêtement entérique et de 15,0 à 35,0 % en poids des agents de revêtement à libération modifiée avec un rapport en poids allant de 5:95 à 30:70 ; et, de manière simultanée, en ajoutant les agents de formation de pores sous forme de poudre, où : le débit de pulvérisation du mélange comprenant les agents de revêtement est de 300 à 1200 mg / min par kg de noyau inerte ; le débit d'addition de solides des agents de formation de pores est de 75 à 500 mg/min par Kg de noyau inerte ; et le rapport du débit de pulvérisation du mélange comprenant les agents de revêtement au débit d'addition des agents de formation de pores sous forme solide est de 90:10 à 60:40 ; et
- préparer la seconde pluralité de pellets à libération modifiée de pyridoxine ou un sel pharmaceutiquement acceptable de celle-ci en revêtant les pellets de pyridoxine ou d'un sel pharmaceutiquement acceptable de celle-ci ayant la couche de revêtement active intérieure en pulvérisant un mélange comprenant de 2,0 à 7,5 % en poids des agents de revêtement entérique et de 15,0 à 35,0 % en poids des agents de revêtement à libération modifiée avec un rapport en poids allant de 5:95 à 30:70 ; et, de manière simultanée, en ajoutant les agents de formation de pores sous forme de poudre, où : le débit de pulvérisation du mélange comprenant les agents de revêtement est de 300 à 1200 mg / min par kg de noyau inerte ; le débit d'addition de solides des agents de formation de pores est de 75 à 500 mg/min par Kg de noyau inerte ; et le rapport du débit de pulvérisation du mélange comprenant les agents de revêtement au débit d'addition des agents de formation de pores sous forme solide est de 90:10 à 60:40.

2. Le procédé selon la revendication 1, dans lequel le procédé comprend :
- préparer la première pluralité de pellets à libération modifiée de doxylamine ou d'un sel pharmaceutiquement acceptable de celle-ci telle que définie dans la revendication 1, dans lequel le débit d'addition de solides des agents de formation de pores est de 88 à 195 mg/min par Kg de noyau inerte ;
et
- préparer la seconde pluralité de pellets à libération modifiée de pyridoxine ou d'un sel pharmaceutiquement acceptable de celle-ci telle que définie dans la revendication 1, dans lequel le débit d'addition de solides des agents de formation de pores est de 193 à 425 mg/min par Kg de noyau inerte.

3. Le procédé selon l'une quelconque des revendications 1 ou 2, dans lequel le procédé comprend :
- préparer la première pluralité de pellets à libération modifiée de doxylamine ou d'un sel pharmaceutiquement acceptable de celle-ci telle que définie dans la revendication 1, dans lequel le rapport du débit de pulvérisation du mélange comprenant les agents de revêtement au débit d'addition de solides des agents de formation de pores est de 90:10 à 60:40 ;
et
- préparer la seconde pluralité de pellets à libération modifiée de pyridoxine ou d'un sel pharmaceutiquement acceptable de celle-ci telle que définie dans la revendication 1, dans lequel le rapport du débit de pulvérisation du mélange comprenant les agents de revêtement au débit d'addition de solides des agents de formation de pores est de 90:10 à 60:40.

4. Le procédé selon l'une quelconque des revendications 1-3, dans lequel le mélange comprend de 2,5 à 5,5 % en poids des agents de revêtement entérique et de 18 à 30 % en poids des agents de revêtement à libération modifiée.

5. Le procédé selon l'une quelconque des revendications 1-4, dans lequel le procédé comprend en outre une étape précédente consistant à revêtir séparément :
- revêtement des pellets de doxylamine ou d'un sel pharmaceutiquement acceptable de celle-ci ayant la couche de revêtement active intérieure en pulvérisant un mélange comprenant de 5 à 15 % des agents de revêtement entérique et, de manière simultanée, ajoutant de 5,5 à 6,0 g par kg de noyau inerte des agents de formation de pores sous forme de poudre ; où le débit de pulvérisation du mélange comprenant de 5 à 15 % en poids d'un ou plusieurs agents de revêtement entérique va de 445 à 1000 mg/min par kg de noyau inerte ; le débit d'addition de solides de l'agent de formation de pores va de 45 à 100 mg/min par Kg de noyau inerte ; et le rapport du débit de pulvérisation du mélange comprenant les agents de revêtement au débit d'addition de solides va de 87:13 à 93:7 ;
et
- revêtement du noyau inerte avec une pulvérisation d'un mélange comprenant de 30 à 45 % d'un ou plusieurs agents de revêtement et, de manière simultanée, l'ajout de la quantité thérapeutiquement efficace de pyridoxine ou d'un sel pharmaceutiquement acceptable de celle-ci sous forme de poudre : où le débit de pulvérisation du mélange comprenant de 30 à 45 % d'un ou plusieurs agents de revêtement va de 385 à 850 mg/min par Kg de noyau inerte ; le débit d'addition de solides de la poudre va de 770 à 1700 mg/min par Kg de noyau inerte ; et le rapport du débit de pulvérisation du mélange comprenant les agents de revêtement au mélange sous forme de poudre va de 25:75 à 40:60.

6. Le procédé selon l'une quelconque des revendications 1-5, dans lequel la taille des particules de doxylamine ou d'un sel pharmaceutiquement acceptable de celle-ci et de pyridoxine ou d'un sel pharmaceutiquement acceptable de celle-ci va de 1 µm à 250 µm.

7. Le procédé selon l'une quelconque des revendications 1-6, dans lequel le procédé comprend en outre une étape additionnelle qui comprend revêtir le noyau inerte avec une pulvérisation d'un mélange comprenant de 30 à 40 % en poids d'un ou plusieurs agents de revêtement et, de manière simultanée, l'ajout d'un mélange sous forme de poudre de la quantité thérapeutiquement efficace de doxylamine ou un sel pharmaceutiquement acceptable de celle-ci allant de 22 à 30 % en poids des agents de formation de poudre et, facultativement, un ou plusieurs excipients pharmaceutiquement acceptables ; où le débit de pulvérisation du mélange comprenant de 30 à 40 % en poids d'un ou plusieurs agents de revêtement va de 445 à 1000 mg/min par Kg de noyau inerte ; le débit d'addition de solides du mélange sous forme de poudre va de 1,64 à 3,62 g/min par Kg de noyau inerte ; et le rapport du débit de pulvérisation du mélange comprenant les agents de revêtement au débit d'addition de solides du mélange sous forme de poudre va de 15:85 à 25:75.

8. Le procédé selon l'une quelconque des revendications 1-7, dans lequel l'agent de formation de pores a une taille des particules allant de 1 µm à 250 µm.

9. Le procédé selon l'une quelconque des revendications 1-8, dans lequel la forme galénique à unités multiples à libération modifiée comprend du succinate de doxylamine et de l'hydrochlorure de pyridoxine.

10. Le procédé selon l'une quelconque des revendications 1-9, dans lequel la forme galénique orale à unités multiples à libération modifiée est une capsule dure comprenant de 5 mg à 50 mg par capsule de succinate de doxylamine et de 5 mg à 50 mg par capsule d'hydrochlorure de pyridoxine.

11. Le procédé selon l'une quelconque des revendications 1-10, dans lequel les agents de revêtement de la couche de revêtement active intérieure de pellets de doxylamine ou d'un sel pharmaceutiquement acceptable de celle-ci sont choisis dans le groupe constitué de la polyvinylpyrrolidone, le shellac, l'hypromellose, l'hydroxypropylcellulose, la cellulose microcristalline et un mélange de ceux-ci ; ou, alternativement,
l'agent de formation de pores est choisi dans le groupe constitué du talc, du sucre micronisé, du chlorure de sodium ou de potassium et un mélange de ceux-ci.

12. Le procédé selon l'une quelconque des revendications 1-11, dans lequel l'agent de revêtement entérique est choisi dans le groupe constitué de copolymère d'acide méthacrylique et méthacrylate de méthyle, copolymère d'acide méthacrylique et acrylate de méthyle, phtalate acétate de cellulose, phtalate d'hydroxypropylméthylcellulose, phtalate acétate de polyvinyle, alginate de sodium, triméllitate d'acétate de cellulose et un mélange de ceux-ci.

13. Le procédé selon l'une quelconque des revendications 1-12, dans lequel le procédé comprend en outre une étape additionnelle consistant à sécher séparément chacune de la pluralité de pellets obtenues dans chacune des étapes de revêtement, dans lequel l'étape de séchage est effectuée à une température allant de 15 °C à 60 °C et avec un débit d'air >1 m³ / (h par kg de noyau inerte) pendant la période de temps appropriée de manière à utiliser moins de 5000 ppm de chacun des solvants dans le procédé.

14. Le procédé selon l'une quelconque des revendications 1-13, dans lequel la forme galénique à unités multiples à libération modifiée présente un profil de dissolution selon lequel :
de 10 % à 35 % en poids de la teneur en doxylamine est dissoute à la 1^{ère} h dans un milieu de HCl 0,1 N (pH = 1) ;
alors, le milieu est remplacé par un milieu de pH = 4,5 (tampon d'acétate 0,05 M) et à la 4^{ème} h une quantité accumulée allant de 45 % à 70 % en poids de la teneur initiale en doxylamine est dissoute ;
alors, le milieu est remplacé par un milieu de pH = 6,8 (tampon de phosphate 0,05 M) et à la 7^{ème} h au moins une quantité accumulée de 80 % de la teneur initiale en doxylamine est dissoute ;
de 10 % à 35 % en poids de la teneur en pyridoxine est dissoute à la 1^{ère} h dans un milieu de HCl 0,1 N (pH = 1) ;
alors, le milieu est remplacé par un milieu de pH = 4,5 (tampon d'acétate 0,05 M) et à la 4^{ème} h une quantité accumulée allant de 40 % à 65 % en poids de la teneur initiale en pyridoxine est dissoute ;
alors, le milieu est remplacé par un milieu de pH = 6,8 (tampon de phosphate 0,05 M) et à la 7^{ème} h au moins une quantité accumulée de 80 % de la teneur initiale en pyridoxine est dissoute ;
dans lequel le profil de dissolution est mesuré en utilisant un appareil USP de type II (« basket »), en situant la composition dans 900 mL du milieu correspondant / tamponné à 37 °C ± 0,5 °C à 100 rpm.

15. Le procédé selon l'une quelconque des revendications 1-14, dans lequel la forme galénique à unités multiples à libération modifiée comprend :
une première pluralité de pellets à libération modifiée de doxylamine ou un sel pharmaceutiquement acceptable de celle-ci comprenant :
- un noyau inerte :
- une couche de revêtement active intérieure comprenant une quantité thérapeutiquement efficace de doxylamine ou un sel pharmaceutiquement acceptable de celle-ci, de 7 à 11 % en poids d'un ou plusieurs agents de revêtement et de 20 à 28 % en poids d'un ou plusieurs agents de formation de pores ; et, facultativement, un ou plusieurs excipients pharmaceutiquement acceptables ;
- une couche de revêtement entérique moyenne comprenant de 45 à 65 % en poids d'un ou plusieurs agents de revêtement entérique et de 55 à 35 % en poids d'un ou plusieurs agents de formation de pores ; et
- une couche de revêtement à libération modifiée extérieure comprenant de 8 à 14 % en poids d'un ou plusieurs agents de revêtement entérique, de 38 à 46 % en poids d'un ou plusieurs agents de revêtement à libération modifiée et de 42 à 52 % en poids d'un ou plusieurs agents de formation de pores ;
et
une seconde pluralité de pellets à libération modifiée de pyridoxine ou un sel pharmaceutiquement acceptable de celle-ci comprenant :
- un noyau inerte :
- une couche de revêtement active intérieure comprenant une quantité thérapeutiquement efficace de pyridoxine ou un sel pharmaceutiquement acceptable de celle-ci, et de 14 à 20 % en poids d'un ou plusieurs agents de revêtement ; et
- une couche de revêtement à libération modifiée extérieure comprenant de 2 à 6 % en poids d'un ou plusieurs agents de revêtement entérique, de 30 à 45 % en poids d'un ou plusieurs agents de revêtement à libération modifiée et de 50 à 65 % en poids d'un ou plusieurs agents de formation de pores.
